# EUROPEAN PATENT APPLICATION

(11) **EP 3 964 266 A1**
(43) Date of publication of application: **09.03.2022**
(21) Application number: 20749226.5
(22) Date of filing: 28.01.2020
(51) Int. Cl.: A61P 43/00, C07K 14/475, C07K 17/10, A61K 47/60, A61K 38/18

(54) **POLYETHYLENE GLYCOL-MODIFIED FORM OF HEPATOCYTE GROWTH FACTOR OR ACTIVE FRAGMENT THEREOF**

(30) Priority: 28.01.2019 JP 2019011807
(71) Applicant: Toray Industries, Inc., Tokyo, 103-8666 (JP)
(72) Inventor: SERIZAWA, Takashi, Kamakura-shi, Kanagawa 248-8555 (JP); NARUMI, Hideki, Kamakura-shi, Kanagawa 248-8555 (JP); MORI, Katsuyuki, Kamakura-shi, Kanagawa 248-8555 (JP); SATO, Mikiya, Kamakura-shi, Kanagawa 248-8555 (JP)
(74) Representative: Kador & Partner PartG mbB
(86) International application number: PCT/JP2020/002863
(87) International publication number: WO 2020/158690

(57) **Abstract**

An objective of the present invention is to provide a polyethylene glycol-modified form of a hepatocyte growth factor or an active fragment thereof which achieves both an *in vivo* half-life extending effect of polyethylene glycol modification and the retainment of bioactivity. The present invention provides a polyethylene glycol-modified form of a hepatocyte growth factor or an active fragment thereof, wherein one molecule of forked-type polyethylene glycol is covalently bound to two molecules of the hepatocyte growth factor or the active fragment thereof at each of their respective carboxyl-terminal regions to form a homodimer.

## Description

### Technical Field

The present invention relates to a polyethylene glycol-modified form of a hepatocyte growth factor or an active fragment thereof.

### Background Art

Hepatocyte growth factor is a growth factor having diverse biological effects and is known to have an anti-apoptotic effect, an angiogenic effect, a vasodilatory effect, an anti-organ fibrosis effect, an anti-epithelial mesenchymal transition effect, and the like, in addition to an originally found hepatocyte proliferative effect. Clinical applications of hepatocyte growth factor to various diseases have been attempted. However, the hepatocyte growth factor needs to be frequently administered in large amounts for sustaining their effects, because the hepatocyte growth factor has an *in vivo* half-life as short as approximately 30 minutes (Non Patent Literature 1).

Natural splicing variants NK1 (Non Patent Literature 2) and NK2 (Non Patent Literature 3) as well as NK4 developed by a gene recombination technique (Non Patent Literature 4) are known as active fragments of the hepatocyte growth factor. These fragments have been found to have bioactivity *in vitro* and *in vivo.*

Polyethylene glycol is a highly biocompatible polymeric macromolecule and is widely used as a protein modifying agent aimed at *in vivo* half-life extension or reduction in immunogenicity for protein medicaments.

Polyethylene glycol-modified forms have also been reported as to the hepatocyte growth factor (Patent Literature 1). A polyethylene glycol-modified form of NK4, an antagonist fragment of the hepatocyte growth factor, has also been reported (Patent Literature 2).

### Citation List

### Patent Literature

Patent Literature 1: US Patent No. 5,977,310 B specification
Patent Literature 2: JP Patent Publication No. 2010-174034 A

### Non Patent Literature

Non Patent Literature 1: Liu K.X. et al., American Journal of Physiology, 1998, Vol. 275, p. 835-842
Non Patent Literature 2: Jakubczak J.L. et al., Molecular and Cellular Biology, 1998, Vol. 18, No. 3, p. 1275-1283
Non Patent Literature 3: Otsuka T. et al., Molecular and Cellular Biology, 2000, Vol. 20, No. 6, p. 2055-2065
Non Patent Literature 4: Date K. et al., FEBS Letters, 1997, Vol. 420, No. 1, p. 1-6

### Summary of Invention

### Technical Problem

Although polyethylene glycol modification has desirable effects, it is known that the polyethylene glycol modification of proteins having bioactivity can cause reduction or loss of the bioactivity, depending on the binding position of polyethylene glycol. For example, although a modified form of a hepatocyte growth factor in which a plurality of molecules of polyethylene glycol are bound to random positions of the hepatocyte growth factor is reported, the *in vivo* half-life extending effect is low and further, 30% or more reduction in bioactivity is observed for the modified form (Patent Literature 1).

Thus, according to conventional techniques, the extension of the *in vivo* half-life by polyethylene glycol modification and the retainment of bioactivity are considered to be incompatible with each other. There is a demand for a polyethylene glycol-modified form that satisfies both of them at once.

Accordingly, an objective of the present invention is to provide a polyethylene glycol-modified form of a hepatocyte growth factor or an active fragment thereof which achieves both an *in vivo* half-life extending effect by polyethylene glycol modification and the retainment of bioactivity.

### Solution to Problem

The present inventors have conducted diligent studies to attain the objective and consequently completed the present invention by finding a polyethylene glycol-modified form of a hepatocyte growth factor or an active fragment thereof which achieves both an *in vivo* half-life extending effect by polyethylene glycol modification and the retainment of bioactivity.

The present invention encompasses the following.
(1) A polyethylene glycol-modified form of a hepatocyte growth factor or an active fragment thereof, wherein one molecule of forked-type polyethylene glycol is covalently bound to two molecules of the hepatocyte growth factor or the active fragment thereof at each of their respective carboxyl-terminal regions to form a homodimer.
(2) The polyethylene glycol-modified form of a hepatocyte growth factor or an active fragment thereof according to (1) above, wherein the polyethylene glycol-modified form is represented by the general formula (I): 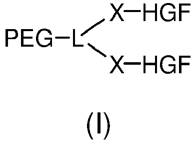 wherein PEG represents a structural moiety of the forked-type polyethylene glycol, L represents a hydrolytically stable branching moiety, HGF represents the hepatocyte growth factor or the active fragment thereof, and X represents a binding moiety that provides a covalent binding of the forked-type polyethylene glycol to the hepatocyte growth factor or the active fragment thereof.
(3) The polyethylene glycol-modified form of a hepatocyte growth factor or an active fragment thereof according to (2) above, wherein in the general formula (I), PEG represents a structure comprising -(CH₂CH₂O)n-, the structure forms a linear structure or a branched structure, and n is 2 to 2300.
(4) The polyethylene glycol-modified form of a hepatocyte growth factor or an active fragment thereof according to any of (1) to (3) above, wherein the polyethylene glycol-modified form is represented by the general formula (II): 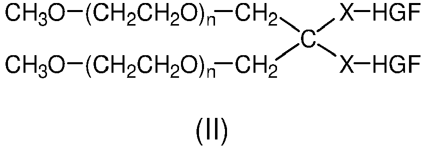 wherein X and HGF are as defined above.
(5) The polyethylene glycol-modified form of a hepatocyte growth factor or an active fragment thereof according to any of (1) to (4) above, wherein a distance between a branch atom of the branching moiety of the forked-type polyethylene glycol and a functional group that provides the covalent binding of the forked-type polyethylene glycol to the hepatocyte growth factor or the active fragment thereof is 20 angstroms or shorter.
(6) The polyethylene glycol-modified form of a hepatocyte growth factor or an active fragment thereof according to any of (1) to (5) above, wherein the active fragment of the hepatocyte growth factor is NK1.
(7) The polyethylene glycol-modified form of a hepatocyte growth factor or an active fragment thereof according to (6) above, wherein the NK1 comprises the amino acid sequence represented by SEQ ID NO: 2 in the sequence listing, or an amino acid sequence having 90% or higher sequence identity to the amino acid sequence.
(8) A medicament comprising the polyethylene glycol-modified form of a hepatocyte growth factor or an active fragment thereof according to any of (1) to (7) above as an active ingredient.

### Advantageous Effects of Invention

The polyethylene glycol-modified form of a hepatocyte growth factor or an active fragment thereof according to the present invention has an extended *in vivo* half-life and also retains its bioactivity and as such, can be used as a medicament that can exert its medicinal effect with less frequency of administration than that of nonmodified forms.

### Brief Description of Drawings

[Figure 1] Figure 1 is a diagram showing an SDS electrophoresis image of a His-Cys-added human NK1 and a forked-type polyethylene glycol-modified NK1 dimer.
[Figure 2] Figure 2 is a diagram showing the bioactivity of a heparin-dependent dimerized NK1, a forked-type polyethylene glycol-modified NK1 dimer and a linear polyethylene glycol-modified NK1 dimer.
[Figure 3] Figure 3 is a diagram showing time course of mouse serum levels of the His-Cys-added human NK1 (dotted line) and forked-type polyethylene glycol-modified NK1 dimer (solid line) when each was administered into the tail vein.

### Description of Embodiments

The polyethylene glycol-modified form of a hepatocyte growth factor or an active fragment thereof according to the present invention is characterized in that the hepatocyte growth factor or the active fragment thereof forms a homodimer, and one molecule of forked-type polyethylene glycol is covalently bound to the carboxyl-terminal regions of both the monomers of the homodimer to form a dimer.

### <Hepatocyte growth factor and active fragment thereof>

The hepatocyte growth factor (hereinafter, also referred to as "HGF") is a growth factor having diverse bioactivities. HGF is composed of N domain, kringle 1, kringle 2, kringle 3, kringle 4 and SPH domain in this order from the amino-terminal side. The N domain, kringle 1, kringle 2, kringle 3 and kringle 4 constitute an α chain, and the SPH domain constitutes a β chain. HGF is biosynthesized as a single-chain pro-HGF and secreted along with the removal of its secretory signal sequence. And then, the resulting sequence is extracellularly processed, between an arginine residue at position 494 (494^{th} arginine residue, or R494) and a valine residue at position 495 (495^{th} valine residue, or V495) counted from the initiating methionine, by protease to form a heterodimer chain of the α chain and the β chain bound through a disulfide bond, which is active (Miyazawa K. et al., The Journal of Biological Chemistry, 1996, Vol. 271, No. 7, p. 3615-3618). The HGF of the present invention means an active HGF having bioactivity.

A human hepatocyte growth factor (HGF) is biosynthesized as a secretory protein consisting of 728 amino acid residues (containing a secretory signal sequence (31 amino acid residues from initiating methionine)) (GenBank accession No. M29145) and, when secreted, becomes a protein consisting of 697 amino acid residues (SEQ ID NO: 1) by the removal of the secretory signal sequence.

The hepatocyte growth factor (HGF) described above encompasses an HGF having the same amino acid sequence as that of naturally occurring HGF (hereinafter, referred to as natural HGF), as well as an amino acid mutant of HGF having an amino acid sequence derived from the amino acid sequence of natural HGF by the deletion, substitution or addition (or insertion) of one or several amino acids and having bioactivity as HGF, and further encompasses even an HGF having an altered sugar chain moiety of natural HGF and an HGF having no sugar chain moiety. The mutant of HGF is preferably a mutant having 90% or higher sequence identity to the amino acid sequence of natural HGF, more preferably a mutant having 95% or higher sequence identity to the amino acid sequence of natural HGF, further preferably a mutant having 98% or higher sequence identity to the amino acid sequence of natural HGF. Examples thereof include a deleted variant of HGF with a deletion of 5 amino acid residues within kringle 1 (which is a naturally occurring mutant) (Kinosaki M. et al., FEBS Letters, 1998, Vol. 434, p. 165-170), which has been reported to have higher specific activity than that of natural HGF in certain cell lines.

The "sequence identity" used in the present specification refers to identity between two sequences that can be determined using an algorithm such as BLAST or FASTA, in which the two sequences are aligned so as to attain the maximum degree of identity with or without gaps, and can generally be calculated as the percentage (%) of the number of identical amino acids to the total number of amino acids (including gaps) (Altschul S. et al., Journal of Molecular Biology, 1990, Vol. 215, No. 3, p. 403-410; and Altschul S. et al., Nucleic Acids Research, 1997, Vol. 25, No. 17, p. 3389-3402).

The term "several" used in the present specification refers to an integer of 2 to 10, i.e., 10, 9, 8, 7, 6, 5, 4, 3, or 2.

As for the "mutant" of a polypeptide used in the present specification, a polypeptide that comprises an amino acid sequence derived from the amino acid sequence of a natural polypeptide (natural hepatocyte growth factor (HGF)) by the deletion, substitution or addition (or insertion) of one or more amino acids to generate a different sequence from the natural polypeptide is referred to as a mutant of the natural polypeptide. Such a mutant may naturally occur or may be artificially produced by use of a common technique such as a gene recombination technique. For artificial production of mutants, it is important not to impair the activity of the polypeptide. For this purpose, it should be noted that, for example: when the mutant is produced, a mutation is introduced neither to an active site of the polypeptide nor, if necessary, to near the active site; substitution is performed on conservative amino acid ; and the HGF activity of the mutant is confirmed by assay.

The conservative amino acid substitution generally refers to substitution between amino acids similar in chemical properties, electric properties (or polarity and/or hydrophobicity) or structural properties. Since such substitution can suppress a marked change in conformation of a polypeptide, the polypeptide can retain its activity without large impairment and, in some times, can have higher activity than the natural one. Specific examples of such amino acid substitution include substitution between acidic amino acids (e.g., aspartic acid (D) and glutamic acid (E)), substitution between basic amino acids (e.g., histidine (H), lysine (K) and arginine (R)), substitution between aromatic amino acids (e.g., phenylalanine (F), tyrosine (Y) and tryptophan (W)), substitution between hydrophilic amino acids (e.g., cysteine (C), aspartic acid (D), glutamic acid (E), histidine (H), lysine (K), asparagine (N), glutamine (Q), arginine (R), serine (S) and threonine (T)), and substitution between hydrophobic amino acids (e.g., alanine (A), phenylalanine (F), isoleucine (I), leucine (L), norleucine (Nle), methionine (M), valine (V), tryptophan (W) and tyrosine (Y)).

The hepatocyte growth factor (HGF) described above also encompasses a recombinant HGF produced by a gene recombination technique on the basis of the amino acid sequence or nucleotide sequence of natural HGF.

It is known that c-Met is a receptor of the hepatocyte growth factor (HGF). The diverse bioactivities of HGF are induced by the binding of the HGF to the HGF receptor, c-Met.

The active fragment of the hepatocyte growth factor (HGF) described above refers to a protein that has a portion of the structure of HGF and exerts bioactivity (agonistic activity) as HGF by binding to an HGF receptor. The active fragment of the HGF described above also encompasses a protein that has a portion of the structure of HGF and acts as an antagonist of HGF (exerts antagonistic activity) by binding to an HGF receptor.

Examples of the active fragment of the hepatocyte growth factor (HGF) include NK1 and NK2 which are natural splicing variants of HGF. NK1 is composed of N domain and kringle 1 on the amino-terminal side of HGF, and NK2 is composed of N domain, kringle 1 and kringle 2 on the amino-terminal side of HGF. It has been reported that each of these fragments acts as an agonist or an antagonist of HGF *in vivo* (Jakubczak J.L. et al., Molecular and Cellular Biology, 1998, Vol. 18, No. 3, p. 1275-1283; and Otsuka T. et al., Molecular and Cellular Biology, 2000, Vol. 20, No. 6, p. 2055-2065).

Another example of the active fragment of the hepatocyte growth factor (HGF) includes NK4 created by a gene recombination technique. NK4 is composed of N domain, kringle 1, kringle 2, kringle 3 and kringle 4 on the amino-terminal side of HGF and has been reported to act as an antagonist of HGF (Date K. et al., FEBS Letters, 1997, Vol. 420, No. 1, p. 1-6).

The active fragment of the hepatocyte growth factor (HGF) described above encompasses an active fragment having the same amino acid sequence derived from the amino acid sequence of natural HGF, as well as a mutant having an amino acid sequence derived from the amino acid sequence of natural HGF by the deletion, substitution or addition of one or several amino acids and having bioactivity (agonistic activity) as HGF or antagonistic activity of HGF, and further encompasses even a mutant having an altered sugar chain moiety derived from natural HGF and a mutant having no sugar chain moiety derived from natural HGF. The mutant of the active fragment of HGF is preferably a mutant having 90% or higher sequence identity to the amino acid sequence of natural HGF, more preferably a mutant having 95% or higher sequence identity to the amino acid sequence of natural HGF, further preferably a mutant having 98% or higher sequence identity to the amino acid sequence of natural HGF. Examples of the highly active NK1 mutant include 1K1 (Lietha D. et al., The EMBO Journal, 2001, Vol. 20, No. 20, p. 5543-5555) and M2.2 (Jones D.S. et al., Proceedings of the National Academy of Sciences of the United States of America, 2011, Vol. 108, No. 32, p. 13035-13040).

The hepatocyte growth factor or the active fragment thereof described above is preferably a hepatocyte growth factor (HGF) (also including a mutant), NK1 (also including a mutant), NK2 (also including a mutant) or NK4 (also including a mutant), preferably a natural HGF, a deleted variant of HGF (Kinosaki M. et al., FEBS Letters, 1998, Vol. 434, p. 165-170), NK1 (also including a mutant), NK2 (also including a mutant) or NK4 (also including a mutant), more preferably NK1 (also including a mutant) or NK2 (also including a mutant), further preferably NK1 (also including a mutant), most preferably human NK1 consisting of the amino acid sequence represented by SEQ ID NO: 2. The amino acid sequence of SEQ ID NO: 2 excludes a secretory signal sequence (MWVTKLLPALLLQHVLLHLLLLPIAIPYAEG: SEQ ID NO: 3) derived from human HGF.

The hepatocyte growth factor or the active fragment thereof described above contains a mammal-derived amino acid sequence, which is preferably a human-, cat- or dog-derived amino acid sequence, more preferably a human-derived amino acid sequence.

An artificial sequence such as a tag sequence may be added to the hepatocyte growth factor or the active fragment thereof described above for the purpose of protein purification or the like. Examples of the tag sequence include a 6 x His tag, HAT tag, c-Myc tag, FLAG tag, DYKDDDDK tag, Strep tag, HA tag, GST tag and MBP tag. Further, an artificial sequence such as a spacer sequence or a protease-cleavable sequence may be inserted between the HGF or the active fragment thereof described above and the tag in order to remove the tag sequence after purification. In this case, the hepatocyte growth factor or the active fragment thereof described above may contain a cleaved fragment of the artificial sequence such as a spacer sequence or a protease-cleavable sequence.

The hepatocyte growth factor or the active fragment thereof described above can also be obtained by use of a method known in the art, such as extraction from a tissue, protein synthesis using a gene recombination technique, or biological production using recombinant cells expressing the hepatocyte growth factor or the active fragment thereof (or natural cells expressing the hepatocyte growth factor). Alternatively, a commercially available hepatocyte growth factor or active fragment thereof may be used as the HGF or the active fragment thereof.

The gene recombination technique can be performed according to a method described in, for example, Molecular Cloning, 2nd ed., 1989, Cold Spring Harbor Laboratory. An exemplary such technique will be described below.

DNA encoding the HGF or the active fragment thereof is provided. The DNA can be obtained by isolating cDNA by selection from a cDNA library prepared from human tissues or cells, and subjecting the cDNA to a DNA amplification method such as PCR. Alternatively, the DNA can be chemically synthesized using, for example, a DNA synthesizer based on a phosphoramidite method.

The DNA described above is incorporated into an appropriate vector to prepare an expression vector. Such a vector contains elements, such as regulatory sequences, necessary for expressing the DNA (and if necessary, for secreting a protein). Specific examples of the elements include a translation start codon and stop codon, a promoter, an enhancer, a terminator, a ribosomal binding site (or a Shine-Dalgarno sequence), a selective marker sequence, and a signal sequence. The necessary elements are inserted in the vector.

A suitable promoter is selected as the promoter according to host cells. Examples of the promoter suitable for cells of *Escherichia* bacteria which are prokaryotes include trp promoter, lac promoter, recA promoter, and λP_{L} promoter. Examples of the promoter suitable for cells of *Bacillus* bacteria include SPO1 promoter, SPO2 promoter, and penP promoter. Examples of the promoter suitable for yeast cells include PHO5 promoter, PGK promoter, GAP promoter, ADH promoter, and AOX promoter. Examples of the promoter suitable for plant cells include cauliflower mosaic virus (CaMV) promoter. Examples of the promoter suitable for insect cells include P10 promoter and polyhedrin promoter. Examples of the promoter suitable for mammalian cells include promoters of viruses such as Rous sarcoma virus, polyoma virus, fowlpox virus, adenovirus, bovine papilloma virus, avian sarcoma virus, cytomegalovirus (SMV), simian virus 40 (SV40), and vaccinia virus, metallothionein promoter, and heat shock promoter.

The selective marker may be, for example, HIS3 gene, LEU2 gene, TRP1 gene, URA3 gene, dihydrofolate reductase gene (methotrexate (MTX) resistance), ampicillin resistance gene, neomycin resistance gene, kanamycin resistance gene, or the like.

A vector suitable for host cells is selected as the expression vector. For example, plasmids, phages, cosmids, virus vectors, and artificial chromosomes (e.g., BAC and YAC) are vectors usually used. Examples of the vector for a prokaryote include *E. coli*-derived plasmids, for example, plasmids of pCR series, plasmids of pBR series, and plasmids of pUC series, and *Bacillus subtilis*-derived plasmids, for example, pUB110, pTP5, and pC194. Examples of the vector for a yeast include yeast-derived plasmids, for example, plasmids of pSH series. Examples of the vector for a plant cell include binary vectors. Examples of the vector for a mammalian cell include commercially available vectors such as pBK-CMV, pcDNA3.1, and pZeoSV (Invitrogen Corp and Stratagene California), and virus vectors (e.g., vectors of adenovirus, adeno-associated virus, pox virus, herpes simplex virus, lentivirus, Sendai virus, vaccinia virus, and SV40).

The host cells are prokaryotic cells such as *E. coli* and *Bacillus subtilis,* or eukaryotic cells such as yeasts, plant cells, and animal cells (e.g., mammalian cells and insect cells). When the host cells are transformed or transfected with the expression vector, an approach known in the art can be used, for example, electroporation, microinjection, cell fusion, DEAE dextran method, calcium phosphate method, particle gun method, or Agrobacterium method.

It is known that the hepatocyte growth factor or the active fragment thereof can be expressed using prokaryotic cells or eukaryotic cells. A recombinant protein can be expressed by transiently or stably in the cells by introducing a nucleic acid (DNA) sequence encoding NK1 protein to the cells. A yeast expression system expresses and secretes a recombinant protein into a culture supernatant and is also suitable for the folding of a protein having a disulfide bond, and further such yeast can be cultured more conveniently than mammalian cells. Thus, the yeast expression system is preferred for the expression of the hepatocyte growth factor or the active fragment thereof having a plurality of intramolecular disulfide bonds.

The "homodimer" in the present specification means a dimer formed by two molecules of a protein having the same amino acid sequence. The "monomer" in the present specification refers to one of the protein molecules constituting the dimer. The hepatocyte growth factor (HGF) exerts its bioactivity by the homodimerization of two molecules of the HGF having the same amino acid sequence and the binding of the homodimer to a HGF receptor (Gherardi E. et al., Proceedings of the National Academy of Sciences of the United States of America, 2006, Vol. 103, No. 11, p. 4046-4051). The active fragment of HGF exerts its bioactivity (agonistic activity) by the homodimerization of two molecules of the active fragment having the same amino acid sequence in the presence of a heparin-like substance and the binding of the homodimer to the HGF receptor (Chirgadze DY. et al., Nature Structural Biology, 1999, Vol. 6, No. 1, p. 72-79). Alternatively, the active fragment of HGF binds as a monomer to the HGF receptor in the absence of the heparin-like substance and thereby exerts antagonistic activity.

For example, the crystal structure (PDB ID: 3MKP) of a dimer of human NK1 (Chirgadze DY. et al., Nature Structural Biology, 1999, Vol. 6, No. 1, p. 72-79) shows that two molecules of human NK1 form a dimer in a bilateral symmetrically paired form, and an amino acid sequence within each kringle structure binds to a sequence within the extracellular domain of the HGF receptor c-Met.

### <Forked-type polyethylene glycol>

Polyethylene glycol (hereinafter, referred to as PEG) is a highly biocompatible polymer. It is known that the binding of PEG to a protein provides effects such as improvement in physical stability, heat stability, resistance to proteolytic enzymes, and solubility, decrease in *in vivo* distribution volume, and improvement in blood circulation, thereby imparting clinical usefulness to the protein (Inada et al., J. Bioact and Compatible Polymers, 1990, Vol. 5, p. 343; Delgado et al., Critical Reviews in Therapeutic Drug Carrier Systems, 1992, Vol. 9, p. 249; and Katre, Advanced Drug Delivery Systems, 1993, Vol. 10, p. 91).

PEG is compatible and includes water-soluble poly(ethylene oxide). Typically, PEG has a repeat unit structure "-(CH₂CH₂O)n-", and in the PEG, the a terminal group or a whole structure of the PEG moiety may vary. The PEG may contain, for example, "-CH₂CH₂-O(CH₂CH₂O)n-CH₂CH₂-" or "-(OCH₂CH₂)nO-", depending on the presence or absence of substitution of the terminal oxygen. Commonly used PEG is a terminally capped PEG. In this terminally capped PEG, the PEG is capped at one of its ends with a relatively inactive group (typically, an alkoxy group such as methoxy (-OCH₃)), whereas the other end has a hydroxyl group or the like that is able to be arbitrarily chemically modified. The PEG may be available as a commercial product, or can be prepared by the ring-opening polymerization of ethylene oxide according to a known method (Sandler and Karo, Polymer Synthesis, Academic Press, New York, Vol. 3, p. 138-161).

The PEG to be used for preparing the PEG-modified form of the hepatocyte growth factor or the active fragment thereof described above is a forked-type PEG. The forked-type PEG is known in the art. Typically, the forked-type PEG has a branching moiety at one of the ends of PEG and two hydroxyl groups (which may further undergo chemical alteration) linked to the branching moiety. The forked-type PEG is described in, for example, International Publication No. WO99/45964. The PEG structural moiety of the forked-type PEG may be linear or branched. Particularly preferably, the structural moiety of the forked-type PEG is branched.

The molecular weight of the forked-type PEG used in the PEG-modified form of the hepatocyte growth factor or the active fragment thereof described above is preferably not less than 5,000 and not more than 240,000, more preferably not less than 10,000 and not more than 80,000, further preferably not less than 20,000 and not more than 45,000, most preferably 20,000. It is known that the *in vivo* half-life extending effect of PEG modification correlates with the molecular weight of PEG (Sundqvist T. et al., Computers and Biomedical Research, 1988, Vol. 21, No. 2, p. 110-116). PEG having a molecular weight of 20000 or more is expected to have a sufficient *in vivo* half-life extending effect. On the other hand, it is known that high-molecular-weight PEG modification reduces distribution in tissues. Therefore, the molecular weight is most preferably 20,000.

One molecule of PEG is composed of many repeat unit structures "-(CH₂CH₂O)n-". In general, the molecular weight of PEG differs among individual molecules and is therefore indicated by an average molecular weight. Thus, the molecular weight of the PEG in the present specification means an average molecular weight.

Depending on a method for binding the PEG to the hepatocyte growth factor or the active fragment thereof described above, it is necessary to activate the PEG at its ends to be used for covalent binding reaction. PEG activated at the ends with a N-hydroxysuccinimide ester, nitrobenzenesulfonate ester, maleimide, o-pyridine disulfide, vinyl sulfone, iodoacetamide, carboxylic acid, azide, phosphine or amine structure can be used. Such activated PEG may be synthesized by a method known in the art. The activated PEG can also be obtained as a commercially available product. Examples of the activated forked-type PEG include SUNBRIGHT^{(R)} PTE2 Series from Yuka Sangyo Co., Ltd. In the present specification, the functional group added to the ends of PEG so as to exhibit binding reactivity with a protein is simply referred to as a "functional group" or a "PEG functional group", and the PEG having such a terminal structure is referred to as "PEG activated with functional group", "activated PEG" or "PEG containing functional group".

For covalently binding of the forked-type PEG to the carboxyl-terminal region of the hepatocyte growth factor or the active fragment thereof described above, the forked-type PEG activated with two functional groups is used. The two functional groups may be two identical functional groups, i.e., homofunctional, or may be two different functional groups, i.e., heterofunctional. The forked-type PEG described above is preferably a forked-type PEG activated with two identical functional groups. More specifically, the forked-type PEG activated with two identical functional groups is preferably represented by the following formula (III).

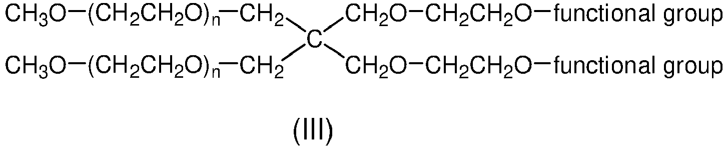

In the formula, "-" represents a bond, and "n" is generally in the range of 2 to approximately 2300. "n" can be appropriately determined depending on the desired PEG molecular weight.

In this context, the binding of PEG to a protein is also referred to as the chemical modification or modification of a protein with PEG, or PEGylation. A covalent conjugate in which PEG is covalently bound to the hepatocyte growth factor or the active fragment thereof is also referred to as a hepatocyte growth factor or an active fragment thereof chemically modified with PEG, or a PEG-modified form of the hepatocyte growth factor or the active fragment thereof.

For the covalent binding of the PEG to the carboxyl-terminal region of the hepatocyte growth factor or the active fragment thereof described above, an amino group (-NH₂), a thiol group (-SH) and a carboxyl group (-COOH) of an amino acid contained in the hepatocyte growth factor or the active fragment thereof, or of a cysteine or a non-natural amino acid artificially introduced into the hepatocyte growth factor or the active fragment thereof by a gene recombination technique known in the art can be used. For example, the amino group is present in side chains of lysine residues at the 4th and 10th residues counted from the carboxyl-terminal amino acid (the most carboxyl-terminal amino acid) of the human hepatocyte growth factor (HGF), and can be selectively modified using a N-hydroxysuccinimide (NHS) ester group as the PEG functional group. Also, for example, if the thiol group is present in side chains of cysteine residues artificially inserted in the carboxyl-terminal regions, the thiol groups in the side chains can be selectively modified using a maleimide group as the PEG functional group since the thiol groups form neither intramolecular nor intermolecular disulfide bonds. Further, for example, the carboxyl group is present in a side chain of aspartic acid at the 8th residue counted from the carboxyl-terminal amino acid, a side chain of glutamic acid at the 2nd residue counted from the carboxyl-terminal amino acid, a side chain of the carboxyl-terminal amino acid glutamic acid, and the carboxyl terminus of human NK1, and can be modified using an amino group or the like. As a method for inserting a non-natural amino acid to the carboxyl-terminal regions of the hepatocyte growth factor or the active fragment thereof, a reported method of introducing azidophenylalanine or azido-Z-lysine, etc. by codon alteration (JP Patent Publication (Kokai) No. 2009-207490 A (2009)) can be used. An azide group contained in such a non-natural amino acid can be selectively modified using triallylphosphine.

For covalently binding PEG to the carboxyl-terminal region of the HGF or the active fragment thereof described above, it is preferred to artificially insert an amino acid that exhibits selective reactivity, into the carboxyl-terminal regions of the hepatocyte growth factor or the active fragment thereof. Particularly, it is more preferred to artificially insert a cysteine residue, an azidophenylalanine residue or an azido-Z-lysine residue into the carboxyl-terminal regions of the hepatocyte growth factor or the active fragment thereof, and it is further preferred to artificially insert a cysteine residue thereto. In this case, PEG activated with a maleimide group as the PEG functional group is preferably used. This permits a site-selective PEG modification at the cysteine residues inserted in the carboxyl-terminal regions of the hepatocyte growth factor or the active fragment thereof.

<PEG-modified form of hepatocyte growth factor or active fragment thereof>

In the PEG-modified form of the hepatocyte growth factor or the active fragment thereof described above, one molecule of the forked-type PEG is covalently bound to the carboxyl-terminal region of each monomer (i.e., each one molecule of the hepatocyte growth factor or the active fragment thereof) constituting the homodimer, thereby forming a dimer. The respective amino termini of the monomers constituting the homodimer of the hepatocyte growth factor or the active fragment thereof described above are located close to each other on the cell membrane side where the hepatocyte growth factor (HGF) receptor is present, while the respective carboxyl termini of the monomers are located close to each other on the intercellular space side. Accordingly, the PEG-modified form of the hepatocyte growth factor or the active fragment thereof described above can retain the bioactivity of the hepatocyte growth factor or the active fragment thereof, because PEG is covalently bound to the carboxyl-terminal regions of the hepatocyte growth factor or the active fragment thereof. The PEG modification of an amino terminus or its neighboring region of the hepatocyte growth factor or the active fragment thereof cannot produce the desired effect.

The carboxyl-terminal regions of the hepatocyte growth factor or the active fragment thereof to which PEG is covalently bound include the carboxyl-terminal amino acid (the most carboxyl-terminal amino acid) or its neighboring region of the hepatocyte growth factor or the active fragment thereof. Specifically, the carboxyl-terminal regions include amino acid residues from the carboxyl-terminal amino acid to the 10th residue counted therefrom. For example, in the human hepatocyte growth factor (HGF) (GenBank accession No: M29145 (SEQ ID NO: 5 (nucleotide sequence), SEQ ID NO: 6 (amino acid sequence)), the carboxyl-terminal region corresponds to a region of amino acid residues from positions 718 to 728 (to the carboxyl-terminal amino acid) counted from the initiating methionine as position 1 in the amino acid sequence of SEQ ID NO: 6. For example, in the human NK1 (ranging from glutamine at position 32 to glutamic acid at position 210 in the amino acid sequence of SEQ ID NO: 6 as shown in GenBank accession No. M29145), the carboxyl-terminal region corresponds to a region of amino acid residues from positions 200 to 210 (to the carboxyl-terminal amino acid) counted from the initiating methionine as position 1 in the amino acid sequence of SEQ ID NO: 6. An amino acid natively contained in the hepatocyte growth factor or the active fragment thereof (also including a mutant) or an artificially introduced amino acid can be used as long as the amino acid residue is positioned in the carboxyl-terminal region. The carboxyl-terminal region of the hepatocyte growth factor or the active fragment thereof is preferably from the carboxyl-terminal amino acid to the 10th residue counted therefrom, more preferably from the carboxyl-terminal amino acid to the 4th residue counted therefrom, most preferably the carboxyl-terminal amino acid.

The covalent binding of PEG to the carboxyl-terminal region of the HGF or the active fragment thereof described above can be carried out by a method known in the art. For example, a method for covalently binding PEG to an amino group of a protein is described in U.S. Patent No. 4917888 and International Publication No. WO 1987/00056. A method for covalently binding PEG to a thiol group of a protein is described in International Publication No. WO 1999/55377. A method for covalently binding PEG to a non-natural amino acid introduced in a protein is described in Bioorganic & Medicinal Chemistry Letters, 2004, Vol. 14, p. 5743-5745.

The purification or concentration of the PEG-modified form of the hepatocyte growth factor or the active fragment thereof described above can be carried out by use of a method known in the art after the covalent binding reaction between the hepatocyte growth factor or the active fragment thereof and the forked-type PEG. The PEG-modified form of the hepatocyte growth factor or the active fragment thereof can be purified or concentrated, for example, by using a method such as chromatography using ion exchange, gel filtration, a hydrophobic support or an affinity support, or a combination thereof so as to remove an unreacted hepatocyte growth factor or active fragment thereof or an unreacted forked-type PEG, or by-products.

The PEG-modified form of the hepatocyte growth factor or the active fragment thereof described above is specifically represented by the following formula (I).

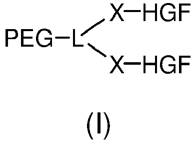

In the formula (I), "-" represents a bond, "PEG" represents a structural moiety of the forked-type polyethylene glycol, "L" represents a hydrolytically stable branching moiety, "X" represents a binding moiety that provides the covalent binding of the forked-type polyethylene glycol to the hepatocyte growth factor or the active fragment thereof, and "HGF" represents the hepatocyte growth factor or the active fragment thereof. The binding (X) of the forked-type polyethylene glycol to the hepatocyte growth factor or the active fragment thereof is hydrolytically stable.

"PEG" is a structural moiety of the forked-type polyethylene glycol. The structure comprising a repeat unit "-(CH₂CH₂O)n-" of polyethylene glycol may form a linear structure or may form a branched structure. Examples of PEG having a branched structure include dibranched, tribranched and tetrabranched PEG. The number of branches of PEG may also include more than four. However, among them, a dibranched PEG is preferred. The branch atom (branch point) of the branching structure may be contained in "L".

"L" may represent a single group such as "-CH-", or may further contain a longer atomic chain (including e.g., an alkylene bond (-CH₂-), an ether bond (-O-), an ester bond (-O-CO- or -CO-O-), an amide bond (-CONH- or -NHCO-) or a combination thereof) on the side of "PEG". Examples of the "L" group include lysine, glycerol, pentaerythritol and sorbitol. A particular branch atom (atom serving as a branch point) in the branching moiety is typically a carbon atom.

"X" is a binding moiety that provides the covalent binding of the forked-type polyethylene glycol to the hepatocyte growth factor or the active fragment thereof, and may represent only an atom derived from the functional group as shown in the formula (III), provided that the distance between the branch atom and the functional group is 20 angstroms (Å) or shorter; or may further contain a longer atomic chain (including e.g., an alkylene bond (-CH₂-), an ether bond (-O-), an ester bond (-O-CO- or -CO-O-), an amide bond (-CONH- or -NHCO-) or a combination thereof) on the side of "L", in addition to the atom derived from the functional group. The appropriate selection of the functional group depends on the binding site to the hepatocyte growth factor or the active fragment thereof, as mentioned above. The corresponding "X" in the obtained PEG-modified form of the hepatocyte growth factor or the active fragment thereof preferably results from the reaction between an appropriate reaction site of the hepatocyte growth factor or the active fragment thereof and the activated forked-type PEG. For example, when the activated forked-type PEG contains an activated ester such as N-hydroxysuccinimide ester, the binding via an amine site of the hepatocyte growth factor or the active fragment thereof forms a corresponding amide bond.

In the present specification, the "functional group" is a reactive group at a terminal portion of the forked-type polyethylene glycol or a terminal portion of the atomic chain described above, for providing a covalent binding to the hepatocyte growth factor or the active fragment thereof.

More specifically, the PEG-modified form of the hepatocyte growth factor or the active fragment thereof described above is preferably represented by the following formula (II).

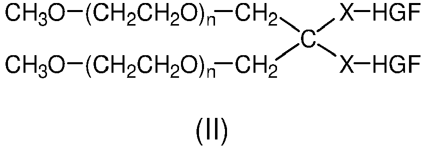

In the formula (II), the definitions of X and HGF are the same as those of the formula (I). The number n of "-(CH₂CH₂O)n-" can be appropriately determined so as to attain the desired PEG molecular weight (described above).

The "hydrolytically stable" binding refers to a chemical bond (typically, covalent binding) that is substantially stable in water (i.e., is not detectably hydrolyzed under physiological conditions over a long time). Examples of the hydrolytically stable binding include a carbon-carbon bond (e.g., within an aliphatic chain), an ether bond, an amide bond, and a urethane bond. In general, the hydrolytically stable binding is a bond that exhibits a hydrolysis rate of less than approximately 1 to 2% per day under physiological conditions.

In the PEG-modified form of the hepatocyte growth factor or the active fragment thereof described above, the distance between the branch atom of the forked-type PEG and the functional group (e.g., a forked-type PEG functional group) that provides the covalent binding of the forked-type polyethylene glycol to the hepatocyte growth factor or the active fragment thereof is not limited as long as the distance allows the PEG-modified form of the hepatocyte growth factor or the active fragment thereof to retain its bioactivity. The distance is preferably 20 angstroms or shorter, more preferably 1.5 to 19 angstroms, further preferably 1.5 to 8 angstroms, most preferably 7.2 angstroms.

The branch atom of the PEG is an atom serving as the branch point, i.e., a branch atom, in "L" in the formula (I). The branch atom is preferably a carbon atom.

The distance between the branch atom of the PEG and the PEG functional group means a distance of the binding site (e.g., five-membered ring nitrogen atom of a maleimide group) of the PEG functional group from the branch atom described above. Specifically, the distance refers to a distance from the branch atom in "L" to the binding site of the functional group in "X" in the formula (I). The distance between the branch atom of the PEG and the PEG functional group can be calculated by, for example, structural simulation. From the viewpoint of the PEG-modified form of the hepatocyte growth factor or the active fragment thereof, the distance between the branch atom of the PEG and the PEG functional group means a distance of half the distance between the respective carboxyl termini of the monomers (i.e., each of which is one molecule of the hepatocyte growth factor or the active fragment thereof) constituting the PEG-modified form of the hepatocyte growth factor or the active fragment thereof (homodimer). When the distance between the branch atom of the PEG and the PEG functional group of the PEG-modified form is, for example, 20 angstroms, the distance between the respective carboxyl termini of the monomers is 40 angstroms.

For retaining the bioactivity of the PEG-modified form of the hepatocyte growth factor or the active fragment thereof described above which forms a homodimer via PEG modification, it is desirable that the distance between the respective carboxyl termini of the monomers (i.e., each of which is one molecule of the hepatocyte growth factor or the active fragment thereof) constituting the dimer should reproduce the crystal structure of the dimer of the hepatocyte growth factor or the active fragment thereof.

In human NK1, a cysteine residue at position 206 (which corresponds to a cysteine residue at position 175 in the amino acid sequence of SEQ ID NO: 2) counted from the initiating methionine at position 1 in the amino acid sequence of SEQ ID NO: 6 forms an intramolecular disulfide bond. A subsequent serine residue at position 207 to glutamic acid at position 210 (which correspond to a serine residue at position 176 to glutamic acid at position 179 in the amino acid sequence of SEQ ID NO: 2) can presumably fluctuate its structure. On the basis of the crystal structure (PDB ID: 3MKP) of a human NK1 dimer (Chirgadze D. Y. et al., Nature Structural Biology, 1999, Vol. 6, No. 1, p. 72-79), the distance between the respective carboxyl termini of the monomers (i.e., each of which is one molecule of human NK1) was structurally simulated, using Molecular Operating Environment (version 2013.08) from Chemical Computing Group, as to the formed dimer of human NK1, on the assumption that the serine residue at position 207 to the glutamic acid at position 210 counted from the initiating methionine at position 1 could be fluctuate its structure. As a result, the minimum distance between the respective carboxyl termini of the monomers was 3 angstroms, and the maximum distance therebetween was 38 angstroms (i.e., the acceptable range of the distance between the branch atom of the PEG and the PEG functional group is 1.5 angstroms at the minimum and 19 angstroms at the maximum).

In the case of adding an artificial sequence such as a tag sequence or a spacer sequence to the carboxyl termini of the hepatocyte growth factor or the active fragment thereof described above, the distance between the respective carboxyl termini of the monomers of the formed dimer of the hepatocyte growth factor or the active fragment thereof may be longer. In the case of adding, for example, a 6 x His tag, to the carboxyl termini of human NK1, a region from the serine residue at position 176 to the glutamic acid at position 179 (serine residue at position 207 to glutamic acid at position 210 counted from the initiating methionine at position 1 in the amino acid sequence of SEQ ID NO: 6) in the amino acid sequence of SEQ ID NO: 2 of human NK1 and further to the 6 x His tag moiety can presumably fluctuate its structure. As a result of carrying out the same structural simulation as above, the distance between the carboxyl termini of the human NK1 dimer was a minimum distance of 3 angstroms and a maximum distance of 88 angstroms (i.e., the acceptable range of the distance between the branch atom of the PEG and the PEG functional group is 1.5 angstroms at the minimum and 44 angstroms at the maximum).

Thus, the distance between the respective carboxyl termini of the monomers in the formed dimer of the PEG-modified form of the hepatocyte growth factor or the active fragment thereof described above is more preferably 3 to 38 angstroms. However, as mentioned above, the distance between the respective carboxyl termini of the monomers may be substantially extended if an artificial sequence is inserted into the carboxyl termini of the hepatocyte growth factor or the active fragment thereof. Examples of the artificial sequence include peptides consisting of 2 to 30 arbitrary amino acids, for example, peptides comprising the tag sequence described above, for example, a peptide of SEQ ID NO: 4 (HHHHHHC).

In the dimerization of the hepatocyte growth factor or the active fragment thereof by PEG modification, it is desirable that, when using a linear PEG activated at both termini with two functional groups, the PEG has the chain length that provides a distance between the functional groups of 40 angstroms or shorter, but the molecular weight of such PEG is not more than 1,000, which therefore cannot produce a sufficient *in vivo* half-life extending effect. On the other hand, in the present invention which uses the forked-type PEG activated with two functional groups, the PEG molecular weight is not limited even though the distance between the branch atom of the PEG and the PEG functional group falls within the preferred range of 20 angstroms or shorter. Furthermore, the modification of the hepatocyte growth factor or the active fragment thereof with the forked-type PEG enables to control the distance between the respective carboxyl termini of the monomers (i.e., each of which is one molecule of the hepatocyte growth factor or the active fragment thereof) constituting the dimer, thereby enabling to maintain the bioactivity of the hepatocyte growth factor or the active fragment thereof even after PEG modification.

A preferred embodiment of the PEG-modified form of the hepatocyte growth factor or the active fragment thereof described above is a PEG-modified form of the hepatocyte growth factor or the active fragment thereof, wherein the hepatocyte growth factor or the active fragment thereof forms a homodimer, and one molecule of forked-type PEG is covalently bound to both the monomers of the homodimer at cysteine residues artificially inserted in the carboxyl-terminal regions of the monomers so as to form a dimer, and the forked-type PEG has a distance of 7.2 angstroms between the branch atom of PEG and the PEG functional group and has a PEG molecular weight of 20,000. This PEG-modified form of the hepatocyte growth factor or the active fragment thereof retains bioactivity because of the controlled distance between the respective carboxyl termini of the monomers constituting the dimer, and further has an *in vivo* half-life extending effect due to the modification with PEG having a molecular weight of 20,000.

In a more preferred embodiment, the PEG-modified form of the hepatocyte growth factor or the active fragment thereof described above is represented by the following formula (IV).

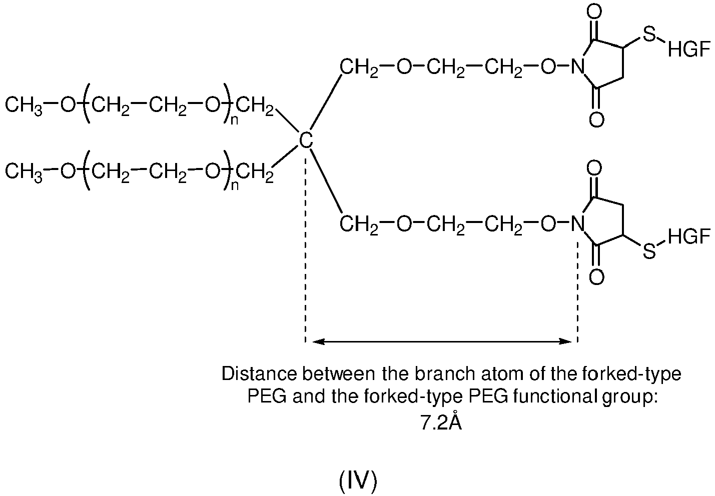

The binding between the hepatocyte growth factor or the active fragment thereof (in the formula, "HGF") and the maleimide group (functional group) is of a "-S-" bond generated by the involvement of the cysteine residues artificially inserted in the carboxyl-terminal regions of the hepatocyte growth factor or the active fragment thereof in the binding. The number n of "-(CH₂CH₂O)n-" is determined depending on the PEG molecular weight (described above).

### <Medicament>

The PEG-modified form of the hepatocyte growth factor or the active fragment thereof described above retains bioactivity inherently possessed by the hepatocyte growth factor or the active fragment thereof and further has an *in vivo* half-life extending effect due to PEG modification and as such can be used as an active ingredient of a medicament (which can be used interchangeably with a "therapeutic drug" or a "pharmaceutical composition").

The bioactivity of the PEG-modified form of the hepatocyte growth factor or the active fragment thereof described above can be conveniently measured using cultured cells. For example, a c-Met phosphorylation-inducing effect, a cell proliferative effect, a cell migration effect and an anti-apoptotic effect which are the effects of the natural hepatocyte growth factor can be used as indexes (Rubin J.S. et al, The Journal of Biological Chemistry, 2001, Vol. 276, No. 35, p. 32977-32983; Lietha D. et al., The EMBO Journal, 2001, Vol. 20, No. 20, p. 5543-5555; and Liu Y. American Journal of Physiology, 1999, Vol. 277, No. 4, p. 624-633).

The *in vivo* half-life of the PEG-modified form of the hepatocyte growth factor or the active fragment thereof described above can be calculated by measuring the concentration in blood of the PEG-modified form intravenously, intraperitoneally, subcutaneously or intradermally administered to experimental animals. Particularly, the radio labeling of the PEG-modified form of the hepatocyte growth factor or the active fragment thereof described above conveniently achieves such measurement.

The medicament comprising the PEG-modified form of the hepatocyte growth factor or the active fragment thereof described above as an active ingredient can be used in the treatment of various diseases that can exploit the bioactivity of the hepatocyte growth factor or the active fragment thereof. The medicament can be used in the treatment of, for example, acute inflammatory disease, chronic inflammatory disease, acute ischemic disease, chronic ischemic disease, amyotrophic lateral sclerosis, organ fibrosis, diabetes mellitus, spinal cord injury, peritoneal adhesion, or neuropathic pain, and further can be used for transplantation therapy or wound healing.

The medicament described above can be used as a useful therapeutic drug for a mammal (e.g., a mouse, a rat, a hamster, a rabbit, a dog, a cat, a monkey, cattle, sheep or a human), particularly, a human. In the case of using the medicament described above as a useful therapeutic drug for a human, the hepatocyte growth factor or the active fragment thereof described above is preferably based on an amino acid sequence derived from a human hepatocyte growth factor.

In the mode of administration of the medicament described above, the PEG-modified form of the hepatocyte growth factor or the active fragment thereof serving as an active ingredient can be orally or parenterally administered, either alone or as a medicament blended with an acceptable carrier. The administration is preferably performed by subcutaneous, intramuscular or intravenous injection.

Examples of the dosage form for the oral administration of the medicament described above include tablets, pills, capsules, granules, syrups, emulsions and suspensions. These dosage forms can be produced by methods known in the art and contain a carrier or an excipient and an optional additive usually used in the pharmaceutical formulation field. Examples of the carrier and the excipient for tablets include lactose, maltose, saccharose, starch and magnesium stearate. Examples of the additive can include binders, disintegrants, preservatives, delayed releasing agents, colorants, flavoring agents, stabilizers, solubilizers, thickeners, and emulsifiers.

Examples of the dosage form for the parenteral administration of the medicament described above include injections, eye drops, ointments, wet dressings, suppositories, transnasal absorption agents, transpulmonary absorption agents, transdermal absorption agents and local sustained-release agents. These dosage forms can be produced by methods known in the art. Solution formulations can be prepared, for example, in a state where the PEG-modified form of the hepatocyte growth factor or the active fragment thereof described above serving as an active ingredient is dissolved in an aseptic aqueous solution for use in injections or suspended and emulsified in extracts, and embedded in liposomes. Solid formulations can be prepared, for example, as freeze-dried products of the PEG-modified form of the hepatocyte growth factor or the active fragment thereof described above serving as an active ingredient supplemented with an excipient such as mannitol, trehalose, sorbitol, lactose or glucose. Such solid formulations may be further pulverized for use. Such powders may be mixed with polylactic acid, glycolic acid, or the like and solidified for use. Gels can be prepared, for example, by dissolving the PEG-modified form of the hepatocyte growth factor or the active fragment thereof described above serving as an active ingredient in a thickener or a polysaccharide such as glycerin, PEG, methylcellulose, carboxymethylcellulose, hyaluronic acid or chondroitin sulfate. If necessary, these formulations can be supplemented with the additive described above.

The medicament described above can generally be administered at 0.001 mg to 100 mg/kg/dosage, preferably 0.01 mg to 10 mg/kg/dosage, in the range of once a month to once a day, preferably once a month to once a week, though the dose is appropriately determined according to the age and body weight of a patient, a disease to be administered, symptoms, the mode of administration, an administration route, the molecular weight of PEG, etc.

Hereinafter, the present invention will be described further specifically with reference to Examples. However, the technical scope of the present invention is not limited by the illustrated Examples.

### Examples

### (Example 1) Expression of human NK1 protein

The expression of the recombinant human NK1 protein was carried out using a commercially available yeast expression kit, Multi-Copy Pichia Expression Kit (Invitrogen Corp.).

DNA encoding the human NK1 protein in which a 6 x His tag and a cysteine residue were added in this order (i.e., HHHHHHC (SEQ ID NO: 4) was added) to the carboxyl terminus of the human NK1 protein without its secretory signal sequence (hereinafter, referred to as His-Cys-added human NK1) was inserted into pPIC9K expression vector included in the Multi-Copy Pichia Expression Kit (Invitrogen Corp.) using In-Fusion HD cloning kit (Takara Bio Inc.). The resulting plasmid for expression was linearized with a restriction enzyme, and then used for gene transfer into GS115 strain included in the kit by electroporation.

The bacterial cells after receiving the gene transfer were selectively cultured for the first stage on a histidine-deficient agar medium. Then, the survived colonies were selectively cultured for the second stage on an agar medium containing 4 mg/mL Geneticin^{(R)} (Roche) to isolate a yeast strain harboring multiple copies of the introduced gene of interest. The isolated strain was precultured in a thermostat bath at 30°C using a BMGY medium, then transferred to BMMY medium containing 0.5% methanol, and main-cultured for 3 days in a thermostat bath at 20°C to induce the expression of the His-Cys-added human NK1, the protein of interest.

### (Example 2) Purification of human NK1 protein

The supernatant of the yeast culture obtained in Example 1 was centrifugally clarified, and then subjected to purification of the His-Cys-added human NK1 in the supernatant using a nickel resin and a heparin resin.

The centrifugally clarified yeast culture supernatant was passed through a heparin resin (GE Healthcare) equilibrated in advance with PBS(-) so that the His-Cys-added human NK1 was attached to the heparin resin. Subsequently, buffers with varying NaCl concentrations in PBS(-) ranging from 150 mM to 2000 mM were passed therethrough in the order of increasing NaCl concentration to obtain each elution fraction. Each elution fraction was subjected to SDS electrophoresis to identify a His-Cys-added human NK1 elution fraction.

Next, the His-Cys-added human NK1 elution fraction obtained by heparin resin purification was passed through cOmplete His-Tag Purification Resin (Roche) equilibrated in advance with PBS(-) containing 300 mM NaCl so that the His-Cys-added human NK1 was attached to the resin. Subsequently, buffers with imidazole (at concentration range: 0 mM to 500 mM) added to PBS(-) containing 300 mM NaCl was passed therethrough in the order of increasing imidazole concentration to obtain each elution fraction. Each elution fraction was subjected to SDS electrophoresis to identify a His-Cys-added human NK1 elution fraction. The obtained His-Cys-added human NK1 elution fraction was concentrated using Amicon Ultra-15 (MWCO of 10000; Merck Millipore) to obtain the His-Cys-added human NK1, the protein of interest.

### (Example 3) Synthesis of PEG-modified NK1 dimer

NK1 dimerized by the covalent binding of one molecule of PEG to the carboxyl termini of two molecules of the His-Cys-added human NK1 (hereinafter, referred to as a PEG-modified NK1 dimer) was synthesized by the following method.

To a solution containing the His-Cys-added human NK1 prepared in Example 2 at the adjusted concentration of 1.0 mg/mL in PBS(-) containing 1.0 mol/L NaCl (pH 7.4) (hereinafter, referred to as an NK1 solution), a solution of 38 mg/mL 2-mercaptoethylamine (hereinafter, referred to as 2-MEA) was added in a 0.07-fold amount, and the mixture was incubated at 37°C for 30 minutes. The incubated NK1 solution was passed through a gel filtration column (Zeba desalt spin column 7 KDa; Thermo Fisher Scientific Inc.) equilibrated with a phosphate buffer (0.3 mol/L NaCl, 0.002 mol/L EDTA, and 0.1 mol/L phosphate (pH 6.0)) to remove 2-MEA from the solution.

To the NK1 solution after the 2-MEA removal treatment, a forked-type PEG (SUNBRIGHT PTE2-200MA2, having two functional groups, PEG functional group being maleimide group, distance between the branch atom of PEG and the PEG functional group being 7.2 Å (angstroms), molecular weight of 20,000; Yuka Sangyo Co., Ltd.) or a linear PEG (SUNBRIGHT DE-100MA, having two functional groups, PEG functional group being maleimide group, distance between the PEG functional groups being approximately 400 Å (angstroms), molecular weight of 10,000; Yuka Sangyo Co., Ltd.) was added at a reaction molar ratio of 5:1 (PEG: His-Cys-added human NK1). The mixture was allowed to react at 25°C for 16 to 18 hours to produce a PEG-modified NK1 dimer. PEG-modified NK1 dimer with a modification with a forked-type PEG is referred to as a forked-type PEG-modified NK1 dimer, and PEG-modified NK1 dimer with a modification with a linear PEG is referred to as a linear PEG-modified NK1 dimer.

The solution after the reaction containing the PEG-modified NK1 dimer was diluted 10-fold with NaCl-free PBS(-) and then passed through an SP support (SP-Sepharose 6 Fast Flow; GE Healthcare) to remove an unreacted PEG reagent from the solution. The PEG-modified NK1 dimer adsorbed on the SP support was eluted with PBS(-) containing 1.0 mol/L NaCl and then concentrated using Amicon Ultra-30 (MWCO of 30,000; Merck Millipore).

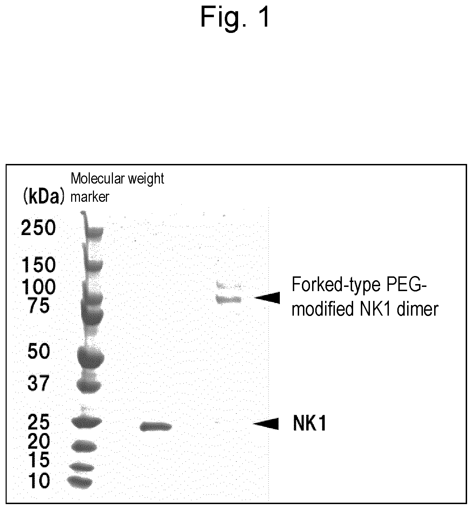

Figure 1 shows an SDS electrophoresis image of the His-Cys-added human NK1 and the forked-type PEG-modified NK1 dimer.

In the forked-type PEG-modified NK1 dimer and the linear PEG-modified NK1 dimer, the dimerization occurs by the covalent binding of one molecule of PEG to the artificially added cysteine residues present at the carboxyl termini of two molecules of the His-Cys-added human NK1. The forked-type PEG-modified NK1 dimer is represented by the following formula (V), and the linear PEG-modified NK1 dimer is represented by the following formula (VI).

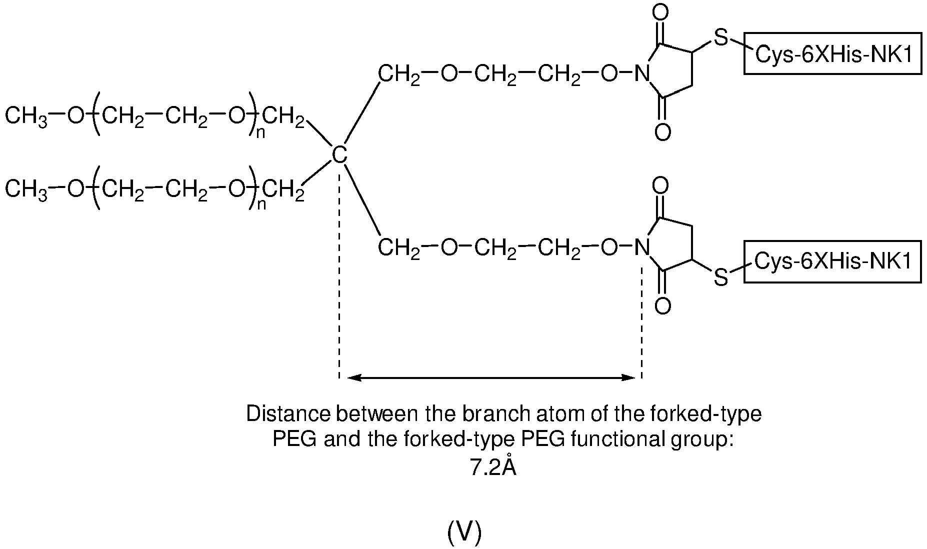

In the formula (V), "Cys-6xHis-NK1" represents the His-Cys-added human NK1, and the thiol group (-SH) of the cysteine residue artificially inserted at each carboxyl terminus of the human NK1 binds to the maleimide group (functional group) to form a "-S-" bond. The number n of "-(CH₂CH₂O)n-" in the formula depends on the PEG molecular weight.

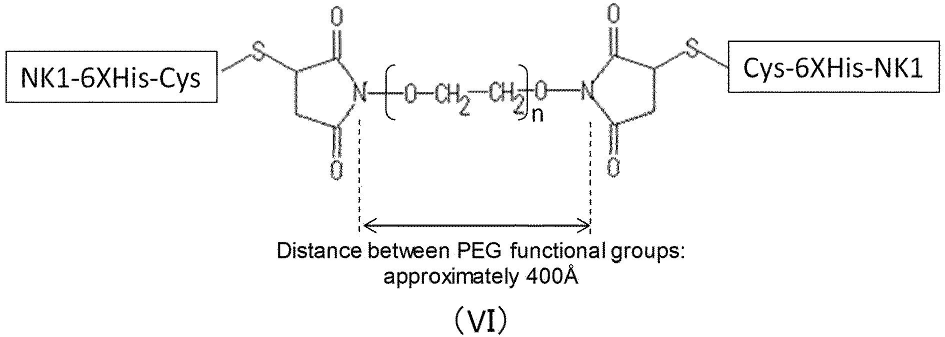

In the formula (VI), "Cys-6xHis-NK1" and "NK1-6xHis-Cys" each represent the His-Cys-added human NK1, and the thiol group (-SH) of the cysteine residue artificially inserted at each carboxyl terminus of the human NK1 binds to the maleimide group (functional group) to form a "-S-" bond. The number n of "-(CH₂CH₂O)n-" in the formula depends on the PEG molecular weight.

### (Example 4) Bioactivity of PEG-modified NK1 dimer

The bioactivity of the PEG-modified NK1 dimer was evaluated by In-Cell ELISA using a phosphorylation inducing activity on HGF receptor present on the cell surface of a human lung epithelial cell line A549 as an index. The His-Cys-added human NK1 used as a comparative control for bioactivity was mixed in advance with MEM medium containing 1 µg/mL purified swine heparin (Sigma-Aldrich Co. LLC) to induce heparin-dependent dimerization, thereby allowing the NK1 to form a dimer (hereinafter, referred to as heparin-dependent dimerized NK1).

A549 cells were suspended in MEM medium containing 10% FCS, seeded at a density of 1.5 × 10⁴ cells/well on a 96-well plate for imaging (Becton, Dickinson and Company), and cultured all night and all day. After reaching 70% confluency of the cells, the medium was replaced with serum-free MEM medium, and the cells were cultured for 16 hours or longer into a serum-starved state. To the cells in the serum-starved state, the heparin-dependent dimerized NK1, the forked-type PEG-modified NK1 dimer or the linear PEG-modified NK1 dimer was added (at 10 or 250 ng/mL), and allowed to react at 37°C for 10 minutes.

The cells were fixed in PBS(-) containing 4% formalin. Subsequently, the cell membranes were permeabilized with PBS(-) containing 0.3% Triton-X and 0.6% hydrogen peroxide. Then, the cells were blocked with 10% BSA. To the cells thus blocked, an anti-phosphorylated c-Met antibody as a primary antibody and a HRP-labeled secondary antibody as a secondary antibody were added, and reacted, followed by the addition of 1 x QuantaRed Enhanced Chemifluorescent HRP Substrate (Thermo Fisher Scientific, Inc.). After incubation at room temperature, fluorescence intensity (RFU) at a wavelength of 590 nm was measured using a plate reader (Envision; PerkinElmer, Inc.) and used as the induced amount of HGF receptor phosphorylation.

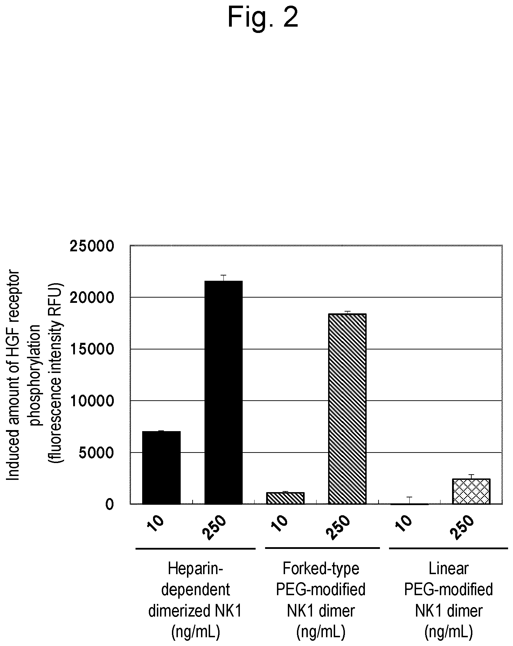

The results are shown in Figure 2. In Figure 2, the ordinate depicts the fluorescence intensity (RFU) that indicates the induced amount of HGF receptor phosphorylation. The abscissa depicts the treatment concentration (ng/mL) of the heparin-dependent dimerized NK1, the forked-type PEG-modified NK1 dimer or the linear PEG-modified NK1 dimer.

The forked-type PEG-modified NK1 dimer retained bioactivity equivalent to that of the heparin-dependent dimerized NK1, whereas the bioactivity of the linear PEG-modified NK1 dimer was considerably diminished as compared with the heparin-dependent dimerized NK1. Thus, for dimerizing NK1 by PEG modification while retaining its bioactivity, it was shown to be important to use a forked-type PEG that enables the distance between the carboxyl termini of NK1 molecules to be controlled.

### (Example 5) Kinetics in blood of forked-type PEG-modified NK1 dimer

The kinetics in blood of the forked-type PEG-modified NK1 dimer were evaluated via administering ^{[125I]} labeled forked-type PEG-modified NK1 dimer to the tail veins of mice. The His-Cys-added human NK1 was used as a comparative control for kinetics in blood.

The ^{[125I]} labeling of the His-Cys-added human NK1 or the forked-type PEG-modified NK1 dimer was performed by the IODO-BEADS method using Na^{[125I]} (Iodine-125 Radionuclide; PerkinElmer, Inc.). The radioactivity of a supernatant and a precipitate after TCA precipitation was measured to verify that the ^{[125I]} labeling rate of the protein to be used for administration was no less than 90%.

The ^{[125I]} labeled His-Cys-added human NK1 or forked-type PEG-modified NK1 dimer was administered (at 25 µg/0.5 MBq/kg) to the tail veins of Crlj:CD1 (ICR) male mice. The serum level was measured at 5 minutes to 24 hours after administration using radioactivity as an index.

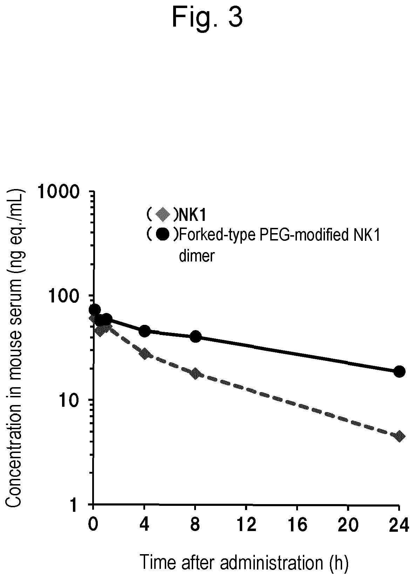

The results are shown in Figure 3. In Figure 3, the ordinate depicts the levels (ng eq./mL) in mouse serum, and the abscissa depicts the time (h) after administration. The dotted line depicts the results of the ^{[125I]} labeled His-Cys-added human NK1, and the solid line depicts the results of the ^{[125I]} labeled forked-type polyethylene glycol-modified NK1 dimer.

The forked-type PEG-modified NK1 dimer exhibited almost the same time course of serum levels as that of the His-Cys-added human NK1 up to 1 hour after administration, but reduction in serum levels of the forked-type PEG-modified NK1 dimer at 4 hours or more after administration was alleviated as compared with that of the His-Cys-added human NK1. Furthermore, at 24 hours after administration, the forked-type PEG-modified NK1 dimer was observed in serum with approximately 4.2 times of the level of the His-Cys-added human NK1. The half-life (t1/2) was 7.9 hours for the administered His-Cys-added human NK1, while it was extended approximately twice to 15.4 hours for the forked-type PEG-modified NK1 dimer. Thus, the forked-type PEG-modified NK1 dimer was shown to exhibit an *in vivo* half-life extending effect, which is an effect of PEG modification.

### Industrial Applicability

The polyethylene glycol-modified form of a hepatocyte growth factor or an active fragment thereof according to the present invention has an extended *in vivo* half-life and retains its bioactivity and as such can be used as a medicament that can exert its medicinal effect with less frequency of administration than that of nonmodified forms.

### Free Text of Sequence Listing

SEQ ID NO: 1: Amino acid sequence of human HGF containing no secretory signal sequence
SEQ ID NO: 2: Amino acid sequence of human NK1 containing no secretory signal sequence
SEQ ID NO: 3: Secretory signal sequence derived from human HGF
SEQ ID NO: 4: Amino acid sequence of a 6 x His tag and a cysteine residue added to the carboxyl terminus of human NK1 protein
SEQ ID NO: 5: Nucleotide sequence of human HGF
SEQ ID NO: 6: Amino acid sequence of human HGF

## Claims

1. A polyethylene glycol-modified form of a hepatocyte growth factor or an active fragment thereof, wherein one molecule of forked-type polyethylene glycol is covalently bound to two molecules of the hepatocyte growth factor or the active fragment thereof at each of their respective carboxyl-terminal regions to form a homodimer.

2. The polyethylene glycol-modified form of a hepatocyte growth factor or an active fragment thereof according to claim 1, wherein the polyethylene glycol-modified form is represented by the general formula (I): 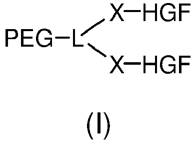 wherein PEG represents a structural moiety of the forked-type polyethylene glycol, L represents a hydrolytically stable branching moiety, HGF represents the hepatocyte growth factor or the active fragment thereof, and X represents a binding moiety that provides a covalent binding of the forked-type polyethylene glycol to the hepatocyte growth factor or the active fragment thereof.

3. The polyethylene glycol-modified form of a hepatocyte growth factor or an active fragment thereof according to claim 2, wherein in the general formula (I), PEG represents a structure comprising -(CH₂CH₂O)n-, the structure forms a linear structure or a branched structure, and n is 2 to 2300.

4. The polyethylene glycol-modified form of a hepatocyte growth factor or an active fragment thereof according to any one of claims 1 to 3, wherein the polyethylene glycol-modified form is represented by the general formula (II): 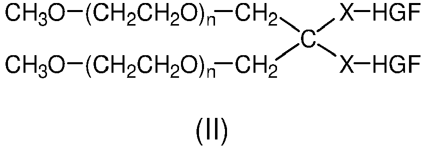 wherein X and HGF are as defined above.

5. The polyethylene glycol-modified form of a hepatocyte growth factor or an active fragment thereof according to any one of claims 1 to 4, wherein a distance between a branch atom of the branching moiety of the forked-type polyethylene glycol and a functional group that provides the covalent binding of the forked-type polyethylene glycol to the hepatocyte growth factor or the active fragment thereof is 20 angstroms or shorter.

6. The polyethylene glycol-modified form of a hepatocyte growth factor or an active fragment thereof according to any one of claims 1 to 5, wherein the active fragment of the hepatocyte growth factor is NK1.

7. The polyethylene glycol-modified form of a hepatocyte growth factor or an active fragment thereof according to claim 6, wherein the NK1 comprises the amino acid sequence represented by SEQ ID NO: 2 in the sequence listing, or an amino acid sequence having 90% or higher sequence identity to the amino acid sequence.

8. A medicament comprising the polyethylene glycol-modified form of a hepatocyte growth factor or an active fragment thereof according to any one of claims 1 to 7 as an active ingredient.


SEQUENCE LISTING

<110>  TORAY INDUSTRIES, INC.

<120>  Hepatocyte growth factor or active fragment thereof modified by
       polyethylene glycol

<130>  19316WO01

<160>  6

<170>  PatentIn version 3.5

<210>  1
<211>  697
<212>  PRT
<213>  Homo sapiens

<220>
<223>  Human HGF amino acid sequence having no secretion signal sequence

<400>  1

Gln Arg Lys Arg Arg Asn Thr Ile His Glu Phe Lys Lys Ser Ala Lys
1               5                   10                  15

Thr Thr Leu Ile Lys Ile Asp Pro Ala Leu Lys Ile Lys Thr Lys Lys
            20                  25                  30

Val Asn Thr Ala Asp Gln Cys Ala Asn Arg Cys Thr Arg Asn Lys Gly
        35                  40                  45

Leu Pro Phe Thr Cys Lys Ala Phe Val Phe Asp Lys Ala Arg Lys Gln
    50                  55                  60

Cys Leu Trp Phe Pro Phe Asn Ser Met Ser Ser Gly Val Lys Lys Glu
65                  70                  75                  80

Phe Gly His Glu Phe Asp Leu Tyr Glu Asn Lys Asp Tyr Ile Arg Asn
                85                  90                  95

Cys Ile Ile Gly Lys Gly Arg Ser Tyr Lys Gly Thr Val Ser Ile Thr
            100                 105                 110

Lys Ser Gly Ile Lys Cys Gln Pro Trp Ser Ser Met Ile Pro His Glu
        115                 120                 125

His Ser Phe Leu Pro Ser Ser Tyr Arg Gly Lys Asp Leu Gln Glu Asn
    130                 135                 140

Tyr Cys Arg Asn Pro Arg Gly Glu Glu Gly Gly Pro Trp Cys Phe Thr
145                 150                 155                 160

Ser Asn Pro Glu Val Arg Tyr Glu Val Cys Asp Ile Pro Gln Cys Ser
                165                 170                 175

Glu Val Glu Cys Met Thr Cys Asn Gly Glu Ser Tyr Arg Gly Leu Met
            180                 185                 190

Asp His Thr Glu Ser Gly Lys Ile Cys Gln Arg Trp Asp His Gln Thr
        195                 200                 205

Pro His Arg His Lys Phe Leu Pro Glu Arg Tyr Pro Asp Lys Gly Phe
    210                 215                 220

Asp Asp Asn Tyr Cys Arg Asn Pro Asp Gly Gln Pro Arg Pro Trp Cys
225                 230                 235                 240

Tyr Thr Leu Asp Pro His Thr Arg Trp Glu Tyr Cys Ala Ile Lys Thr
                245                 250                 255

Cys Ala Asp Asn Thr Met Asn Asp Thr Asp Val Pro Leu Glu Thr Thr
            260                 265                 270

Glu Cys Ile Gln Gly Gln Gly Glu Gly Tyr Arg Gly Thr Val Asn Thr
        275                 280                 285

Ile Trp Asn Gly Ile Pro Cys Gln Arg Trp Asp Ser Gln Tyr Pro His
    290                 295                 300

Glu His Asp Met Thr Pro Glu Asn Phe Lys Cys Lys Asp Leu Arg Glu
305                 310                 315                 320

Asn Tyr Cys Arg Asn Pro Asp Gly Ser Glu Ser Pro Trp Cys Phe Thr
                325                 330                 335

Thr Asp Pro Asn Ile Arg Val Gly Tyr Cys Ser Gln Ile Pro Asn Cys
            340                 345                 350

Asp Met Ser His Gly Gln Asp Cys Tyr Arg Gly Asn Gly Lys Asn Tyr
        355                 360                 365

Met Gly Asn Leu Ser Gln Thr Arg Ser Gly Leu Thr Cys Ser Met Trp
    370                 375                 380

Asp Lys Asn Met Glu Asp Leu His Arg His Ile Phe Trp Glu Pro Asp
385                 390                 395                 400

Ala Ser Lys Leu Asn Glu Asn Tyr Cys Arg Asn Pro Asp Asp Asp Ala
                405                 410                 415

His Gly Pro Trp Cys Tyr Thr Gly Asn Pro Leu Ile Pro Trp Asp Tyr
            420                 425                 430

Cys Pro Ile Ser Arg Cys Glu Gly Asp Thr Thr Pro Thr Ile Val Asn
        435                 440                 445

Leu Asp His Pro Val Ile Ser Cys Ala Lys Thr Lys Gln Leu Arg Val
    450                 455                 460

Val Asn Gly Ile Pro Thr Arg Thr Asn Ile Gly Trp Met Val Ser Leu
465                 470                 475                 480

Arg Tyr Arg Asn Lys His Ile Cys Gly Gly Ser Leu Ile Lys Glu Ser
                485                 490                 495

Trp Val Leu Thr Ala Arg Gln Cys Phe Pro Ser Arg Asp Leu Lys Asp
            500                 505                 510

Tyr Glu Ala Trp Leu Gly Ile His Asp Val His Gly Arg Gly Asp Glu
        515                 520                 525

Lys Cys Lys Gln Val Leu Asn Val Ser Gln Leu Val Tyr Gly Pro Glu
    530                 535                 540

Gly Ser Asp Leu Val Leu Met Lys Leu Ala Arg Pro Ala Val Leu Asp
545                 550                 555                 560

Asp Phe Val Ser Thr Ile Asp Leu Pro Asn Tyr Gly Cys Thr Ile Pro
                565                 570                 575

Glu Lys Thr Ser Cys Ser Val Tyr Gly Trp Gly Tyr Thr Gly Leu Ile
            580                 585                 590

Asn Tyr Asp Gly Leu Leu Arg Val Ala His Leu Tyr Ile Met Gly Asn
        595                 600                 605

Glu Lys Cys Ser Gln His His Arg Gly Lys Val Thr Leu Asn Glu Ser
    610                 615                 620

Glu Ile Cys Ala Gly Ala Glu Lys Ile Gly Ser Gly Pro Cys Glu Gly
625                 630                 635                 640

Asp Tyr Gly Gly Pro Leu Val Cys Glu Gln His Lys Met Arg Met Val
                645                 650                 655

Leu Gly Val Ile Val Pro Gly Arg Gly Cys Ala Ile Pro Asn Arg Pro

Gly Ile Phe Val Arg Val Ala Tyr Tyr Ala Lys Trp Ile His Lys Ile
        675                 680                 685

Ile Leu Thr Tyr Lys Val Pro Gln Ser
    690                 695

<210>  2
<211>  179
<212>  PRT
<213>  Homo sapiens

<220>
<223>  Human NK1 amino acid sequence having no secretion signal sequence

<400>  2

Gln Arg Lys Arg Arg Asn Thr Ile His Glu Phe Lys Lys Ser Ala Lys
1               5                   10                  15

Thr Thr Leu Ile Lys Ile Asp Pro Ala Leu Lys Ile Lys Thr Lys Lys
            20                  25                  30

Val Asn Thr Ala Asp Gln Cys Ala Asn Arg Cys Thr Arg Asn Lys Gly
        35                  40                  45

Leu Pro Phe Thr Cys Lys Ala Phe Val Phe Asp Lys Ala Arg Lys Gln
    50                  55                  60

Cys Leu Trp Phe Pro Phe Asn Ser Met Ser Ser Gly Val Lys Lys Glu
65                  70                  75                  80

Phe Gly His Glu Phe Asp Leu Tyr Glu Asn Lys Asp Tyr Ile Arg Asn
                85                  90                  95

Cys Ile Ile Gly Lys Gly Arg Ser Tyr Lys Gly Thr Val Ser Ile Thr
            100                 105                 110

Lys Ser Gly Ile Lys Cys Gln Pro Trp Ser Ser Met Ile Pro His Glu
        115                 120                 125

His Ser Phe Leu Pro Ser Ser Tyr Arg Gly Lys Asp Leu Gln Glu Asn
    130                 135                 140

Tyr Cys Arg Asn Pro Arg Gly Glu Glu Gly Gly Pro Trp Cys Phe Thr
145                 150                 155                 160

Ser Asn Pro Glu Val Arg Tyr Glu Val Cys Asp Ile Pro Gln Cys Ser
                165                 170                 175

Glu Val Glu

<210> 3
<211> 31
<212> PRT
<213> Homo sapiens

<220>
<223> Human HGF-derived secretion signal sequence

<400> 3

Met Trp Val Thr Lys Leu Leu Pro Ala Leu Leu Leu Gln His Val Leu
1               5                   10                  15

Leu His Leu Leu Leu Leu Pro Ile Ala Ile Pro Tyr Ala Glu Gly
            20                  25                  30

<210> 4
<211> 7
<212> PRT
<213> Artificial

<220>
<223> Amino acid sequence of 6xHis tag and cysteine residue, added to
the carboxyl terminus of human NK1 protein

<400> 4

His His His His His His Cys
1               5

<210> 5
<211> 2576
<212> DNA
<213> Homo sapiens

<220>
<221> CDS
<222> (102)..(2288)

<400> 5
gggctcagag ccgactggct cttttaggca ctgactccga acaggattct ttcacccagg 60

catctcctcc agagggatcc gccagcccgt ccagcagcac c atg tgg gtg acc aaa 116
Met Trp Val Thr Lys
1               5

ctc ctg cca gcc ctg ctg ctg cag cat gtc ctc ctg cat ctc ctc ctg 164
Leu Leu Pro Ala Leu Leu Leu Gln His Val Leu Leu His Leu Leu Leu
                10                  15                  20

ctc ccc atc gcc atc ccc tat gca gag gga caa agg aaa aga aga aat 212
Leu Pro Ile Ala Ile Pro Tyr Ala Glu Gly Gln Arg Lys Arg Arg Asn
            25                  30                  35

aca att cat gaa ttc aaa aaa tca gca aag act acc cta atc aaa ata 260
Thr Ile His Glu Phe Lys Lys Ser Ala Lys Thr Thr Leu Ile Lys Ile
        40                  45                  50

gat cca gca ctg aag ata aaa acc aaa aaa gtg aat act gca gac caa 308
Asp Pro Ala Leu Lys Ile Lys Thr Lys Lys Val Asn Thr Ala Asp Gln
    55                  60                  65

tgt gct aat aga tgt act agg aat aaa gga ctt cca ttc act tgc aag 356
Cys Ala Asn Arg Cys Thr Arg Asn Lys Gly Leu Pro Phe Thr Cys Lys
70                  75                  80                  85

gct ttt gtt ttt gat aaa gca aga aaa caa tgc ctc tgg ttc ccc ttc 404
Ala Phe Val Phe Asp Lys Ala Arg Lys Gln Cys Leu Trp Phe Pro Phe
                90                  95                  100

aat agc atg tca agt gga gtg aaa aaa gaa ttt ggc cat gaa ttt gac 452
Asn Ser Met Ser Ser Gly Val Lys Lys Glu Phe Gly His Glu Phe Asp
            105                 110                 115

ctc tat gaa aac aaa gac tac att aga aac tgc atc att ggt aaa gga 500
Leu Tyr Glu Asn Lys Asp Tyr Ile Arg Asn Cys Ile Ile Gly Lys Gly
        120                 125                 130

cgc agc tac aag gga aca gta tct atc act aag agt ggc atc aaa tgt 548
Arg Ser Tyr Lys Gly Thr Val Ser Ile Thr Lys Ser Gly Ile Lys Cys
    135                 140                 145

cag ccc tgg agt tcc atg ata cca cac gaa cac agc ttt ttg cct tcg 596
Gln Pro Trp Ser Ser Met Ile Pro His Glu His Ser Phe Leu Pro Ser
150                 155                 160                 165

agc tat cgg ggt aaa gac cta cag gaa aac tac tgt cga aat cct cga 644
Ser Tyr Arg Gly Lys Asp Leu Gln Glu Asn Tyr Cys Arg Asn Pro Arg
                170                 175                 180

ggg gaa gaa ggg gga ccc tgg tgt ttc aca agc aat cca gag gta cgc 692
Gly Glu Glu Gly Gly Pro Trp Cys Phe Thr Ser Asn Pro Glu Val Arg
            185                 190                 195

tac gaa gtc tgt gac att cct cag tgt tca gaa gtt gaa tgc atg acc 740
Tyr Glu Val Cys Asp Ile Pro Gln Cys Ser Glu Val Glu Cys Met Thr
        200                 205                 210

tgc aat ggg gag agt tat cga ggt ctc atg gat cat aca gaa tca ggc 788
Cys Asn Gly Glu Ser Tyr Arg Gly Leu Met Asp His Thr Glu Ser Gly
    215                 220                 225

aag att tgt cag cgc tgg gat cat cag aca cca cac cgg cac aaa ttc 836
Lys Ile Cys Gln Arg Trp Asp His Gln Thr Pro His Arg His Lys Phe
230                 235                 240                 245

ttg cct gaa aga tat ccc gac aag ggc ttt gat gat aat tat tgc cgc 884
Leu Pro Glu Arg Tyr Pro Asp Lys Gly Phe Asp Asp Asn Tyr Cys Arg
                250                 255                 260

aat ccc gat ggc cag ccg agg cca tgg tgc tat act ctt gac cct cac 932
Asn Pro Asp Gly Gln Pro Arg Pro Trp Cys Tyr Thr Leu Asp Pro His
            265                 270                 275

acc cgc tgg gag tac tgt gca att aaa aca tgc gct gac aat act atg 980
Thr Arg Trp Glu Tyr Cys Ala Ile Lys Thr Cys Ala Asp Asn Thr Met
        280                 285                 290

aat gac act gat gtt cct ttg gaa aca act gaa tgc atc caa ggt caa 1028
Asn Asp Thr Asp Val Pro Leu Glu Thr Thr Glu Cys Ile Gln Gly Gln
    295                 300                 305

gga gaa ggc tac agg ggc act gtc aat acc att tgg aat gga att cca 1076
Gly Glu Gly Tyr Arg Gly Thr Val Asn Thr Ile Trp Asn Gly Ile Pro
310                 315                 320                 325

tgt cag cgt tgg gat tct cag tat cct cac gag cat gac atg act cct 1124
Cys Gln Arg Trp Asp Ser Gln Tyr Pro His Glu His Asp Met Thr Pro
                330                 335                 340

gaa aat ttc aag tgc aag gac cta cga gaa aat tac tgc cga aat cca 1172
Glu Asn Phe Lys Cys Lys Asp Leu Arg Glu Asn Tyr Cys Arg Asn Pro
            345                 350                 355

gat ggg tct gaa tca ccc tgg tgt ttt acc act gat cca aac atc cga 1220
Asp Gly Ser Glu Ser Pro Trp Cys Phe Thr Thr Asp Pro Asn Ile Arg
        360                 365                 370

gtt ggc tac tgc tcc caa att cca aac tgt gat atg tca cat gga caa 1268
Val Gly Tyr Cys Ser Gln Ile Pro Asn Cys Asp Met Ser His Gly Gln
    375                 380                 385

gat tgt tat cgt ggg aat ggc aaa aat tat atg ggc aac tta tcc caa 1316
Asp Cys Tyr Arg Gly Asn Gly Lys Asn Tyr Met Gly Asn Leu Ser Gln
390                 395                 400                 405

aca aga tct gga cta aca tgt tca atg tgg gac aag aac atg gaa gac 1364
Thr Arg Ser Gly Leu Thr Cys Ser Met Trp Asp Lys Asn Met Glu Asp
                410                 415                 420

tta cat cgt cat atc ttc tgg gaa cca gat gca agt aag ctg aat gag 1412
Leu His Arg His Ile Phe Trp Glu Pro Asp Ala Ser Lys Leu Asn Glu
            425                 430                 435

aat tac tgc cga aat cca gat gat gat gct cat gga ccc tgg tgc tac 1460
Asn Tyr Cys Arg Asn Pro Asp Asp Asp Ala His Gly Pro Trp Cys Tyr
        440                 445                 450

acg gga aat cca ctc att cct tgg gat tat tgc cct att tct cgt tgt 1508
Thr Gly Asn Pro Leu Ile Pro Trp Asp Tyr Cys Pro Ile Ser Arg Cys
    455                 460                 465

gaa ggt gat acc aca cct aca ata gtc aat tta gac cat ccc gta ata 1556
Glu Gly Asp Thr Thr Pro Thr Ile Val Asn Leu Asp His Pro Val Ile
470                 475                 480                 485

tct tgt gcc aaa acg aaa caa ttg cga gtt gta aat ggg att cca aca 1604
Ser Cys Ala Lys Thr Lys Gln Leu Arg Val Val Asn Gly Ile Pro Thr
                490                 495                 500

cga aca aac ata gga tgg atg gtt agt ttg aga tac aga aat aaa cat 1652
Arg Thr Asn Ile Gly Trp Met Val Ser Leu Arg Tyr Arg Asn Lys His
            505                 510                 515

atc tgc gga gga tca ttg ata aag gag agt tgg gtt ctt act gca cga 1700
Ile Cys Gly Gly Ser Leu Ile Lys Glu Ser Trp Val Leu Thr Ala Arg
        520                 525                 530

cag tgt ttc cct tct cga gac ttg aaa gat tat gaa gct tgg ctt gga 1748
Gln Cys Phe Pro Ser Arg Asp Leu Lys Asp Tyr Glu Ala Trp Leu Gly

535                 540                 545

att cat gat gtc cac gga aga gga gat gag aaa tgc aaa cag gtt ctc      1796
Ile His Asp Val His Gly Arg Gly Asp Glu Lys Cys Lys Gln Val Leu
550                 555                 560                 565

aat gtt tcc cag ctg gta tat ggc cct gaa gga tca gat ctg gtt tta      1844
Asn Val Ser Gln Leu Val Tyr Gly Pro Glu Gly Ser Asp Leu Val Leu
                570                 575                 580

atg aag ctt gcc agg cct gct gtc ctg gat gat ttt gtt agt acg att      1892
Met Lys Leu Ala Arg Pro Ala Val Leu Asp Asp Phe Val Ser Thr Ile
            585                 590                 595

gat tta cct aat tat gga tgc aca att cct gaa aag acc agt tgc agt      1940
Asp Leu Pro Asn Tyr Gly Cys Thr Ile Pro Glu Lys Thr Ser Cys Ser
        600                 605                 610

gtt tat ggc tgg ggc tac act gga ttg atc aac tat gat ggc cta tta      1988
Val Tyr Gly Trp Gly Tyr Thr Gly Leu Ile Asn Tyr Asp Gly Leu Leu
    615                 620                 625

cga gtg gca cat ctc tat ata atg gga aat gag aaa tgc agc cag cat      2036
Arg Val Ala His Leu Tyr Ile Met Gly Asn Glu Lys Cys Ser Gln His
630                 635                 640                 645

cat cga ggg aag gtg act ctg aat gag tct gaa ata tgt gct ggg gct      2084
His Arg Gly Lys Val Thr Leu Asn Glu Ser Glu Ile Cys Ala Gly Ala
                650                 655                 660

gaa aag att gga tca gga cca tgt gag ggg gat tat ggt ggc cca ctt      2132
Glu Lys Ile Gly Ser Gly Pro Cys Glu Gly Asp Tyr Gly Gly Pro Leu
            665                 670                 675

gtt tgt gag caa cat aaa atg aga atg gtt ctt ggt gtc att gtt cct      2180
Val Cys Glu Gln His Lys Met Arg Met Val Leu Gly Val Ile Val Pro
        680                 685                 690

ggt cgt gga tgt gcc att cca aat cgt cct ggt att ttt gtc cga gta      2228
Gly Arg Gly Cys Ala Ile Pro Asn Arg Pro Gly Ile Phe Val Arg Val
    695                 700                 705

gca tat tat gca aaa tgg ata cac aaa att att tta aca tat aag gta      2276
Ala Tyr Tyr Ala Lys Trp Ile His Lys Ile Ile Leu Thr Tyr Lys Val
710                 715                 720                 725

cca cag tca tag ctgaagtaag tgtgtctgaa gcacccacca atacaactgt      2328
Pro Gln Ser

cttttacatg aagatttcag agaatgtgga atttaaaatg tcacttacaa caatcctaag      2388

acaactactg gagagtcatg tttgttgaaa ttctcattaa tgtttatggg tgttttctgt      2448

tgttttgttt gtcagtgtta ttttgtcaat gttgaagtga attaaggtac atgcaagtgt      2508

aataacatat ctcctgaaga tacttgaatg gattaaaaaa acacacaggt atatttgctg      2568

gatgataa                                                              2576

<210>  6
<211>  728

<212>   PRT
<213>   Homo sapiens

<400>   6

Met Trp Val Thr Lys Leu Leu Pro Ala Leu Leu Leu Gln His Val Leu
1               5                   10                  15

Leu His Leu Leu Leu Leu Pro Ile Ala Ile Pro Tyr Ala Glu Gly Gln
            20                  25                  30

Arg Lys Arg Arg Asn Thr Ile His Glu Phe Lys Lys Ser Ala Lys Thr
        35                  40                  45

Thr Leu Ile Lys Ile Asp Pro Ala Leu Lys Ile Lys Thr Lys Lys Val
    50                  55                  60

Asn Thr Ala Asp Gln Cys Ala Asn Arg Cys Thr Arg Asn Lys Gly Leu
65                  70                  75                  80

Pro Phe Thr Cys Lys Ala Phe Val Phe Asp Lys Ala Arg Lys Gln Cys
                85                  90                  95

Leu Trp Phe Pro Phe Asn Ser Met Ser Ser Gly Val Lys Lys Glu Phe
            100                 105                 110

Gly His Glu Phe Asp Leu Tyr Glu Asn Lys Asp Tyr Ile Arg Asn Cys
        115                 120                 125

Ile Ile Gly Lys Gly Arg Ser Tyr Lys Gly Thr Val Ser Ile Thr Lys
    130                 135                 140

Ser Gly Ile Lys Cys Gln Pro Trp Ser Ser Met Ile Pro His Glu His
145                 150                 155                 160

Ser Phe Leu Pro Ser Ser Tyr Arg Gly Lys Asp Leu Gln Glu Asn Tyr
                165                 170                 175

Cys Arg Asn Pro Arg Gly Glu Glu Gly Gly Pro Trp Cys Phe Thr Ser
            180                 185                 190

Asn Pro Glu Val Arg Tyr Glu Val Cys Asp Ile Pro Gln Cys Ser Glu
        195                 200                 205

Val Glu Cys Met Thr Cys Asn Gly Glu Ser Tyr Arg Gly Leu Met Asp
    210                 215                 220

His Thr Glu Ser Gly Lys Ile Cys Gln Arg Trp Asp His Gln Thr Pro
225                 230                 235                 240

His Arg His Lys Phe Leu Pro Glu Arg Tyr Pro Asp Lys Gly Phe Asp
                245                 250                 255

Asp Asn Tyr Cys Arg Asn Pro Asp Gly Gln Pro Arg Pro Trp Cys Tyr
            260                 265                 270

Thr Leu Asp Pro His Thr Arg Trp Glu Tyr Cys Ala Ile Lys Thr Cys
        275                 280                 285

Ala Asp Asn Thr Met Asn Asp Thr Asp Val Pro Leu Glu Thr Thr Glu
    290                 295                 300

Cys Ile Gln Gly Gln Gly Glu Gly Tyr Arg Gly Thr Val Asn Thr Ile
305                 310                 315                 320

Trp Asn Gly Ile Pro Cys Gln Arg Trp Asp Ser Gln Tyr Pro His Glu
                325                 330                 335

His Asp Met Thr Pro Glu Asn Phe Lys Cys Lys Asp Leu Arg Glu Asn
            340                 345                 350

Tyr Cys Arg Asn Pro Asp Gly Ser Glu Ser Pro Trp Cys Phe Thr Thr
        355                 360                 365

Asp Pro Asn Ile Arg Val Gly Tyr Cys Ser Gln Ile Pro Asn Cys Asp
    370                 375                 380

Met Ser His Gly Gln Asp Cys Tyr Arg Gly Asn Gly Lys Asn Tyr Met
385                 390                 395                 400

Gly Asn Leu Ser Gln Thr Arg Ser Gly Leu Thr Cys Ser Met Trp Asp
                405                 410                 415

Lys Asn Met Glu Asp Leu His Arg His Ile Phe Trp Glu Pro Asp Ala
            420                 425                 430

Ser Lys Leu Asn Glu Asn Tyr Cys Arg Asn Pro Asp Asp Asp Ala His
        435                 440                 445

Gly Pro Trp Cys Tyr Thr Gly Asn Pro Leu Ile Pro Trp Asp Tyr Cys
    450                 455                 460

Pro Ile Ser Arg Cys Glu Gly Asp Thr Thr Pro Thr Ile Val Asn Leu
465                 470                 475                 480

Asp His Pro Val Ile Ser Cys Ala Lys Thr Lys Gln Leu Arg Val Val

                485                 490                 495

Asn Gly Ile Pro Thr Arg Thr Asn Ile Gly Trp Met Val Ser Leu Arg
            500                 505                 510

Tyr Arg Asn Lys His Ile Cys Gly Gly Ser Leu Ile Lys Glu Ser Trp
        515                 520                 525

Val Leu Thr Ala Arg Gln Cys Phe Pro Ser Arg Asp Leu Lys Asp Tyr
    530                 535                 540

Glu Ala Trp Leu Gly Ile His Asp Val His Gly Arg Gly Asp Glu Lys
545                 550                 555                 560

Cys Lys Gln Val Leu Asn Val Ser Gln Leu Val Tyr Gly Pro Glu Gly
                565                 570                 575

Ser Asp Leu Val Leu Met Lys Leu Ala Arg Pro Ala Val Leu Asp Asp
            580                 585                 590

Phe Val Ser Thr Ile Asp Leu Pro Asn Tyr Gly Cys Thr Ile Pro Glu
        595                 600                 605

Lys Thr Ser Cys Ser Val Tyr Gly Trp Gly Tyr Thr Gly Leu Ile Asn
    610                 615                 620

Tyr Asp Gly Leu Leu Arg Val Ala His Leu Tyr Ile Met Gly Asn Glu
625                 630                 635                 640

Lys Cys Ser Gln His His Arg Gly Lys Val Thr Leu Asn Glu Ser Glu
                645                 650                 655

Ile Cys Ala Gly Ala Glu Lys Ile Gly Ser Gly Pro Cys Glu Gly Asp
            660                 665                 670

Tyr Gly Gly Pro Leu Val Cys Glu Gln His Lys Met Arg Met Val Leu
        675                 680                 685

Gly Val Ile Val Pro Gly Arg Gly Cys Ala Ile Pro Asn Arg Pro Gly
    690                 695                 700

Ile Phe Val Arg Val Ala Tyr Tyr Ala Lys Trp Ile His Lys Ile Ile
705                 710                 715                 720

Leu Thr Tyr Lys Val Pro Gln Ser
                725

SEQUENCE LISTING

<110>  TORAY INDUSTRIES, INC.

<120>  Hepatocyte growth factor or active fragment thereof modified by
       polyethylene glycol

<130>  19316WO01

<160>  7

<170>  PatentIn version 3.5

<210>  1
<211>  697
<212>  PRT
<213>  Homo sapiens

<220>
<223>  Human HGF amino acid sequence having no secretion signal sequence

<400>  1

Gln Arg Lys Arg Arg Asn Thr Ile His Glu Phe Lys Lys Ser Ala Lys
1               5                   10                  15

Thr Thr Leu Ile Lys Ile Asp Pro Ala Leu Lys Ile Lys Thr Lys Lys
            20                  25                  30

Val Asn Thr Ala Asp Gln Cys Ala Asn Arg Cys Thr Arg Asn Lys Gly
        35                  40                  45

Leu Pro Phe Thr Cys Lys Ala Phe Val Phe Asp Lys Ala Arg Lys Gln
    50                  55                  60

Cys Leu Trp Phe Pro Phe Asn Ser Met Ser Ser Gly Val Lys Lys Glu
65                  70                  75                  80

Phe Gly His Glu Phe Asp Leu Tyr Glu Asn Lys Asp Tyr Ile Arg Asn
                85                  90                  95

Cys Ile Ile Gly Lys Gly Arg Ser Tyr Lys Gly Thr Val Ser Ile Thr
            100                 105                 110

Lys Ser Gly Ile Lys Cys Gln Pro Trp Ser Ser Met Ile Pro His Glu
        115                 120                 125

His Ser Phe Leu Pro Ser Ser Tyr Arg Gly Lys Asp Leu Gln Glu Asn
    130                 135                 140

Tyr Cys Arg Asn Pro Arg Gly Glu Glu Gly Gly Pro Trp Cys Phe Thr
145                 150                 155                 160

Ser Asn Pro Glu Val Arg Tyr Glu Val Cys Asp Ile Pro Gln Cys Ser
                165                 170                 175

Glu Val Glu Cys Met Thr Cys Asn Gly Glu Ser Tyr Arg Gly Leu Met
            180                 185                 190

Asp His Thr Glu Ser Gly Lys Ile Cys Gln Arg Trp Asp His Gln Thr
        195                 200                 205

Pro His Arg His Lys Phe Leu Pro Glu Arg Tyr Pro Asp Lys Gly Phe
    210                 215                 220

Asp Asp Asn Tyr Cys Arg Asn Pro Asp Gly Gln Pro Arg Pro Trp Cys
225                 230                 235                 240

Tyr Thr Leu Asp Pro His Thr Arg Trp Glu Tyr Cys Ala Ile Lys Thr
                245                 250                 255

Cys Ala Asp Asn Thr Met Asn Asp Thr Asp Val Pro Leu Glu Thr Thr
            260                 265                 270

Glu Cys Ile Gln Gly Gln Gly Glu Gly Tyr Arg Gly Thr Val Asn Thr
        275                 280                 285

Ile Trp Asn Gly Ile Pro Cys Gln Arg Trp Asp Ser Gln Tyr Pro His
    290                 295                 300

Glu His Asp Met Thr Pro Glu Asn Phe Lys Cys Lys Asp Leu Arg Glu
305                 310                 315                 320

Asn Tyr Cys Arg Asn Pro Asp Gly Ser Glu Ser Pro Trp Cys Phe Thr
                325                 330                 335

Thr Asp Pro Asn Ile Arg Val Gly Tyr Cys Ser Gln Ile Pro Asn Cys
            340                 345                 350

Asp Met Ser His Gly Gln Asp Cys Tyr Arg Gly Asn Gly Lys Asn Tyr
        355                 360                 365

Met Gly Asn Leu Ser Gln Thr Arg Ser Gly Leu Thr Cys Ser Met Trp
    370                 375                 380

Asp Lys Asn Met Glu Asp Leu His Arg His Ile Phe Trp Glu Pro Asp
385                 390                 395                 400

Ala Ser Lys Leu Asn Glu Asn Tyr Cys Arg Asn Pro Asp Asp Asp Ala
                405                 410                 415

His Gly Pro Trp Cys Tyr Thr Gly Asn Pro Leu Ile Pro Trp Asp Tyr
            420                 425                 430

Cys Pro Ile Ser Arg Cys Glu Gly Asp Thr Thr Pro Thr Ile Val Asn
        435                 440                 445

Leu Asp His Pro Val Ile Ser Cys Ala Lys Thr Lys Gln Leu Arg Val
    450                 455                 460

Val Asn Gly Ile Pro Thr Arg Thr Asn Ile Gly Trp Met Val Ser Leu
465                 470                 475                 480

Arg Tyr Arg Asn Lys His Ile Cys Gly Gly Ser Leu Ile Lys Glu Ser
                485                 490                 495

Trp Val Leu Thr Ala Arg Gln Cys Phe Pro Ser Arg Asp Leu Lys Asp
            500                 505                 510

Tyr Glu Ala Trp Leu Gly Ile His Asp Val His Gly Arg Gly Asp Glu
        515                 520                 525

Lys Cys Lys Gln Val Leu Asn Val Ser Gln Leu Val Tyr Gly Pro Glu
    530                 535                 540

Gly Ser Asp Leu Val Leu Met Lys Leu Ala Arg Pro Ala Val Leu Asp
545                 550                 555                 560

Asp Phe Val Ser Thr Ile Asp Leu Pro Asn Tyr Gly Cys Thr Ile Pro
                565                 570                 575

Glu Lys Thr Ser Cys Ser Val Tyr Gly Trp Gly Tyr Thr Gly Leu Ile
            580                 585                 590

Asn Tyr Asp Gly Leu Leu Arg Val Ala His Leu Tyr Ile Met Gly Asn
        595                 600                 605

Glu Lys Cys Ser Gln His His Arg Gly Lys Val Thr Leu Asn Glu Ser
    610                 615                 620

Glu Ile Cys Ala Gly Ala Glu Lys Ile Gly Ser Gly Pro Cys Glu Gly
625                 630                 635                 640

Asp Tyr Gly Gly Pro Leu Val Cys Glu Gln His Lys Met Arg Met Val
                645                 650                 655

Leu Gly Val Ile Val Pro Gly Arg Gly Cys Ala Ile Pro Asn Arg Pro
            660                 665                 670

Gly Ile Phe Val Arg Val Ala Tyr Tyr Ala Lys Trp Ile His Lys Ile
        675                 680                 685

Ile Leu Thr Tyr Lys Val Pro Gln Ser
    690                 695

<210>  2
<211>  179
<212>  PRT
<213>  Homo sapiens

<220>
<223>  Human NK1 amino acid sequence having no secretion signal sequence

<400>  2

Gln Arg Lys Arg Arg Asn Thr Ile His Glu Phe Lys Lys Ser Ala Lys
1               5                   10                  15

Thr Thr Leu Ile Lys Ile Asp Pro Ala Leu Lys Ile Lys Thr Lys Lys
            20                  25                  30

Val Asn Thr Ala Asp Gln Cys Ala Asn Arg Cys Thr Arg Asn Lys Gly
        35                  40                  45

Leu Pro Phe Thr Cys Lys Ala Phe Val Phe Asp Lys Ala Arg Lys Gln
    50                  55                  60

Cys Leu Trp Phe Pro Phe Asn Ser Met Ser Ser Gly Val Lys Lys Glu
65                  70                  75                  80

Phe Gly His Glu Phe Asp Leu Tyr Glu Asn Lys Asp Tyr Ile Arg Asn
                85                  90                  95

Cys Ile Ile Gly Lys Gly Arg Ser Tyr Lys Gly Thr Val Ser Ile Thr
            100                 105                 110

Lys Ser Gly Ile Lys Cys Gln Pro Trp Ser Ser Met Ile Pro His Glu
        115                 120                 125

His Ser Phe Leu Pro Ser Ser Tyr Arg Gly Lys Asp Leu Gln Glu Asn
    130                 135                 140

Tyr Cys Arg Asn Pro Arg Gly Glu Glu Gly Gly Pro Trp Cys Phe Thr
145                 150                 155                 160

Ser Asn Pro Glu Val Arg Tyr Glu Val Cys Asp Ile Pro Gln Cys Ser
                165                 170                 175

Glu Val Glu

<210> 3
<211> 31
<212> PRT
<213> Homo sapiens

<220>
<223> Human HGF-derived secretion signal sequence

<400> 3

Met Trp Val Thr Lys Leu Leu Pro Ala Leu Leu Leu Gln His Val Leu
1               5                   10                  15

Leu His Leu Leu Leu Leu Pro Ile Ala Ile Pro Tyr Ala Glu Gly
            20                  25                  30

<210> 4
<211> 7
<212> PRT
<213> Artificial

<220>
<223> Amino acid sequence of 6xHis tag and cysteine residue, added to
the carboxyl terminus of human NK1 protein

<400> 4

His His His His His His Cys
1               5

<210> 5
<211> 2576
<212> DNA
<213> Homo sapiens

<220>
<221> CDS
<222> (102)..(2288)

<400> 5
gggctcagag ccgactggct cttttaggca ctgactccga acaggattct ttcacccagg 60

catctcctcc agagggatcc gccagcccgt ccagcagcac c atg tgg gtg acc aaa 116
Met Trp Val Thr Lys
1               5

ctc ctg cca gcc ctg ctg ctg cag cat gtc ctc ctg cat ctc ctc ctg 164
Leu Leu Pro Ala Leu Leu Leu Gln His Val Leu Leu His Leu Leu Leu
                10                  15                  20

ctc ccc atc gcc atc ccc tat gca gag gga caa agg aaa aga aga aat 212
Leu Pro Ile Ala Ile Pro Tyr Ala Glu Gly Gln Arg Lys Arg Arg Asn
            25                  30                  35

aca att cat gaa ttc aaa aaa tca gca aag act acc cta atc aaa ata 260
Thr Ile His Glu Phe Lys Lys Ser Ala Lys Thr Thr Leu Ile Lys Ile
        40                  45                  50

gat cca gca ctg aag ata aaa acc aaa aaa gtg aat act gca gac caa 308
Asp Pro Ala Leu Lys Ile Lys Thr Lys Lys Val Asn Thr Ala Asp Gln
    55                  60                  65

tgt gct aat aga tgt act agg aat aaa gga ctt cca ttc act tgc aag 356
Cys Ala Asn Arg Cys Thr Arg Asn Lys Gly Leu Pro Phe Thr Cys Lys
70                  75                  80                  85

gct ttt gtt ttt gat aaa gca aga aaa caa tgc ctc tgg ttc ccc ttc 404
Ala Phe Val Phe Asp Lys Ala Arg Lys Gln Cys Leu Trp Phe Pro Phe
                90                  95                  100

aat agc atg tca agt gga gtg aaa aaa gaa ttt ggc cat gaa ttt gac 452
Asn Ser Met Ser Ser Gly Val Lys Lys Glu Phe Gly His Glu Phe Asp
            105                 110                 115

ctc tat gaa aac aaa gac tac att aga aac tgc atc att ggt aaa gga 500
Leu Tyr Glu Asn Lys Asp Tyr Ile Arg Asn Cys Ile Ile Gly Lys Gly
        120                 125                 130

cgc agc tac aag gga aca gta tct atc act aag agt ggc atc aaa tgt 548
Arg Ser Tyr Lys Gly Thr Val Ser Ile Thr Lys Ser Gly Ile Lys Cys
    135                 140                 145

cag ccc tgg agt tcc atg ata cca cac gaa cac agc ttt ttg cct tcg 596
Gln Pro Trp Ser Ser Met Ile Pro His Glu His Ser Phe Leu Pro Ser
150                 155                 160                 165

agc tat cgg ggt aaa gac cta cag gaa aac tac tgt cga aat cct cga 644
Ser Tyr Arg Gly Lys Asp Leu Gln Glu Asn Tyr Cys Arg Asn Pro Arg
                170                 175                 180

ggg gaa gaa ggg gga ccc tgg tgt ttc aca agc aat cca gag gta cgc 692
Gly Glu Glu Gly Gly Pro Trp Cys Phe Thr Ser Asn Pro Glu Val Arg
            185                 190                 195

tac gaa gtc tgt gac att cct cag tgt tca gaa gtt gaa tgc atg acc 740
Tyr Glu Val Cys Asp Ile Pro Gln Cys Ser Glu Val Glu Cys Met Thr
        200                 205                 210

tgc aat ggg gag agt tat cga ggt ctc atg gat cat aca gaa tca ggc 788
Cys Asn Gly Glu Ser Tyr Arg Gly Leu Met Asp His Thr Glu Ser Gly
    215                 220                 225

aag att tgt cag cgc tgg gat cat cag aca cca cac cgg cac aaa ttc 836
Lys Ile Cys Gln Arg Trp Asp His Gln Thr Pro His Arg His Lys Phe
230                 235                 240                 245

ttg cct gaa aga tat ccc gac aag ggc ttt gat gat aat tat tgc cgc 884
Leu Pro Glu Arg Tyr Pro Asp Lys Gly Phe Asp Asp Asn Tyr Cys Arg
                250                 255                 260

aat ccc gat ggc cag ccg agg cca tgg tgc tat act ctt gac cct cac 932
Asn Pro Asp Gly Gln Pro Arg Pro Trp Cys Tyr Thr Leu Asp Pro His
            265                 270                 275

acc cgc tgg gag tac tgt gca att aaa aca tgc gct gac aat act atg 980
Thr Arg Trp Glu Tyr Cys Ala Ile Lys Thr Cys Ala Asp Asn Thr Met
        280                 285                 290

aat gac act gat gtt cct ttg gaa aca act gaa tgc atc caa ggt caa 1028
Asn Asp Thr Asp Val Pro Leu Glu Thr Thr Glu Cys Ile Gln Gly Gln
    295                 300                 305

gga gaa ggc tac agg ggc act gtc aat acc att tgg aat gga att cca 1076
Gly Glu Gly Tyr Arg Gly Thr Val Asn Thr Ile Trp Asn Gly Ile Pro
310                 315                 320                 325

tgt cag cgt tgg gat tct cag tat cct cac gag cat gac atg act cct 1124
Cys Gln Arg Trp Asp Ser Gln Tyr Pro His Glu His Asp Met Thr Pro
                330                 335                 340

gaa aat ttc aag tgc aag gac cta cga gaa aat tac tgc cga aat cca 1172
Glu Asn Phe Lys Cys Lys Asp Leu Arg Glu Asn Tyr Cys Arg Asn Pro
            345                 350                 355

gat ggg tct gaa tca ccc tgg tgt ttt acc act gat cca aac atc cga 1220
Asp Gly Ser Glu Ser Pro Trp Cys Phe Thr Thr Asp Pro Asn Ile Arg
        360                 365                 370

gtt ggc tac tgc tcc caa att cca aac tgt gat atg tca cat gga caa 1268
Val Gly Tyr Cys Ser Gln Ile Pro Asn Cys Asp Met Ser His Gly Gln
    375                 380                 385

gat tgt tat cgt ggg aat ggc aaa aat tat atg ggc aac tta tcc caa 1316
Asp Cys Tyr Arg Gly Asn Gly Lys Asn Tyr Met Gly Asn Leu Ser Gln
390                 395                 400                 405

aca aga tct gga cta aca tgt tca atg tgg gac aag aac atg gaa gac 1364
Thr Arg Ser Gly Leu Thr Cys Ser Met Trp Asp Lys Asn Met Glu Asp
                410                 415                 420

tta cat cgt cat atc ttc tgg gaa cca gat gca agt aag ctg aat gag 1412
Leu His Arg His Ile Phe Trp Glu Pro Asp Ala Ser Lys Leu Asn Glu
            425                 430                 435

aat tac tgc cga aat cca gat gat gat gct cat gga ccc tgg tgc tac 1460
Asn Tyr Cys Arg Asn Pro Asp Asp Asp Ala His Gly Pro Trp Cys Tyr
        440                 445                 450

acg gga aat cca ctc att cct tgg gat tat tgc cct att tct cgt tgt 1508
Thr Gly Asn Pro Leu Ile Pro Trp Asp Tyr Cys Pro Ile Ser Arg Cys
    455                 460                 465

gaa ggt gat acc aca cct aca ata gtc aat tta gac cat ccc gta ata 1556
Glu Gly Asp Thr Thr Pro Thr Ile Val Asn Leu Asp His Pro Val Ile
470                 475                 480                 485

tct tgt gcc aaa acg aaa caa ttg cga gtt gta aat ggg att cca aca 1604
Ser Cys Ala Lys Thr Lys Gln Leu Arg Val Val Asn Gly Ile Pro Thr
                490                 495                 500

cga aca aac ata gga tgg atg gtt agt ttg aga tac aga aat aaa cat 1652
Arg Thr Asn Ile Gly Trp Met Val Ser Leu Arg Tyr Arg Asn Lys His
            505                 510                 515

atc tgc gga gga tca ttg ata aag gag agt tgg gtt ctt act gca cga 1700
Ile Cys Gly Gly Ser Leu Ile Lys Glu Ser Trp Val Leu Thr Ala Arg
        520                 525                 530

cag tgt ttc cct tct cga gac ttg aaa gat tat gaa gct tgg ctt gga 1748
Gln Cys Phe Pro Ser Arg Asp Leu Lys Asp Tyr Glu Ala Trp Leu Gly

535                 540                 545

att cat gat gtc cac gga aga gga gat gag aaa tgc aaa cag gtt ctc      1796
Ile His Asp Val His Gly Arg Gly Asp Glu Lys Cys Lys Gln Val Leu
550                 555                 560                 565

aat gtt tcc cag ctg gta tat ggc cct gaa gga tca gat ctg gtt tta      1844
Asn Val Ser Gln Leu Val Tyr Gly Pro Glu Gly Ser Asp Leu Val Leu
                570                 575                 580

atg aag ctt gcc agg cct gct gtc ctg gat gat ttt gtt agt acg att      1892
Met Lys Leu Ala Arg Pro Ala Val Leu Asp Asp Phe Val Ser Thr Ile
            585                 590                 595

gat tta cct aat tat gga tgc aca att cct gaa aag acc agt tgc agt      1940
Asp Leu Pro Asn Tyr Gly Cys Thr Ile Pro Glu Lys Thr Ser Cys Ser
        600                 605                 610

gtt tat ggc tgg ggc tac act gga ttg atc aac tat gat ggc cta tta      1988
Val Tyr Gly Trp Gly Tyr Thr Gly Leu Ile Asn Tyr Asp Gly Leu Leu
    615                 620                 625

cga gtg gca cat ctc tat ata atg gga aat gag aaa tgc agc cag cat      2036
Arg Val Ala His Leu Tyr Ile Met Gly Asn Glu Lys Cys Ser Gln His
630                 635                 640                 645

cat cga ggg aag gtg act ctg aat gag tct gaa ata tgt gct ggg gct      2084
His Arg Gly Lys Val Thr Leu Asn Glu Ser Glu Ile Cys Ala Gly Ala
                650                 655                 660

gaa aag att gga tca gga cca tgt gag ggg gat tat ggt ggc cca ctt      2132
Glu Lys Ile Gly Ser Gly Pro Cys Glu Gly Asp Tyr Gly Gly Pro Leu
            665                 670                 675

gtt tgt gag caa cat aaa atg aga atg gtt ctt ggt gtc att gtt cct      2180
Val Cys Glu Gln His Lys Met Arg Met Val Leu Gly Val Ile Val Pro
        680                 685                 690

ggt cgt gga tgt gcc att cca aat cgt cct ggt att ttt gtc cga gta      2228
Gly Arg Gly Cys Ala Ile Pro Asn Arg Pro Gly Ile Phe Val Arg Val
    695                 700                 705

gca tat tat gca aaa tgg ata cac aaa att att tta aca tat aag gta      2276
Ala Tyr Tyr Ala Lys Trp Ile His Lys Ile Ile Leu Thr Tyr Lys Val
710                 715                 720                 725

cca cag tca tag ctgaagtaag tgtgtctgaa gcacccacca atacaactgt      2328
Pro Gln Ser

cttttacatg aagatttcag agaatgtgga atttaaaatg tcacttacaa caatcctaag      2388

acaactactg gagagtcatg tttgttgaaa ttctcattaa tgtttatggg tgttttctgt      2448

tgttttgttt gtcagtgtta ttttgtcaat gttgaagtga attaaggtac atgcaagtgt      2508

aataacatat ctcctgaaga tacttgaatg gattaaaaaa acacacaggt atatttgctg      2568

gatgataa                                                              2576

<210>  6
<211>  728

<212>   PRT
<213>   Homo sapiens

<400>   6

Met Trp Val Thr Lys Leu Leu Pro Ala Leu Leu Leu Gln His Val Leu
1               5                   10                  15

Leu His Leu Leu Leu Leu Pro Ile Ala Ile Pro Tyr Ala Glu Gly Gln
            20                  25                  30

Arg Lys Arg Arg Asn Thr Ile His Glu Phe Lys Lys Ser Ala Lys Thr
        35                  40                  45

Thr Leu Ile Lys Ile Asp Pro Ala Leu Lys Ile Lys Thr Lys Lys Val
    50                  55                  60

Asn Thr Ala Asp Gln Cys Ala Asn Arg Cys Thr Arg Asn Lys Gly Leu
65                  70                  75                  80

Pro Phe Thr Cys Lys Ala Phe Val Phe Asp Lys Ala Arg Lys Gln Cys
                85                  90                  95

Leu Trp Phe Pro Phe Asn Ser Met Ser Ser Gly Val Lys Lys Glu Phe
            100                 105                 110

Gly His Glu Phe Asp Leu Tyr Glu Asn Lys Asp Tyr Ile Arg Asn Cys
        115                 120                 125

Ile Ile Gly Lys Gly Arg Ser Tyr Lys Gly Thr Val Ser Ile Thr Lys
    130                 135                 140

Ser Gly Ile Lys Cys Gln Pro Trp Ser Ser Met Ile Pro His Glu His
145                 150                 155                 160

Ser Phe Leu Pro Ser Ser Tyr Arg Gly Lys Asp Leu Gln Glu Asn Tyr
                165                 170                 175

Cys Arg Asn Pro Arg Gly Glu Glu Gly Gly Pro Trp Cys Phe Thr Ser
            180                 185                 190

Asn Pro Glu Val Arg Tyr Glu Val Cys Asp Ile Pro Gln Cys Ser Glu
        195                 200                 205

Val Glu Cys Met Thr Cys Asn Gly Glu Ser Tyr Arg Gly Leu Met Asp
    210                 215                 220

His Thr Glu Ser Gly Lys Ile Cys Gln Arg Trp Asp His Gln Thr Pro
225                 230                 235                 240

His Arg His Lys Phe Leu Pro Glu Arg Tyr Pro Asp Lys Gly Phe Asp
                245                 250                 255

Asp Asn Tyr Cys Arg Asn Pro Asp Gly Gln Pro Arg Pro Trp Cys Tyr
            260                 265                 270

Thr Leu Asp Pro His Thr Arg Trp Glu Tyr Cys Ala Ile Lys Thr Cys
        275                 280                 285

Ala Asp Asn Thr Met Asn Asp Thr Asp Val Pro Leu Glu Thr Thr Glu
    290                 295                 300

Cys Ile Gln Gly Gln Gly Glu Gly Tyr Arg Gly Thr Val Asn Thr Ile
305                 310                 315                 320

Trp Asn Gly Ile Pro Cys Gln Arg Trp Asp Ser Gln Tyr Pro His Glu
                325                 330                 335

His Asp Met Thr Pro Glu Asn Phe Lys Cys Lys Asp Leu Arg Glu Asn
            340                 345                 350

Tyr Cys Arg Asn Pro Asp Gly Ser Glu Ser Pro Trp Cys Phe Thr Thr
        355                 360                 365

Asp Pro Asn Ile Arg Val Gly Tyr Cys Ser Gln Ile Pro Asn Cys Asp
    370                 375                 380

Met Ser His Gly Gln Asp Cys Tyr Arg Gly Asn Gly Lys Asn Tyr Met
385                 390                 395                 400

Gly Asn Leu Ser Gln Thr Arg Ser Gly Leu Thr Cys Ser Met Trp Asp
                405                 410                 415

Lys Asn Met Glu Asp Leu His Arg His Ile Phe Trp Glu Pro Asp Ala
            420                 425                 430

Ser Lys Leu Asn Glu Asn Tyr Cys Arg Asn Pro Asp Asp Asp Ala His
        435                 440                 445

Gly Pro Trp Cys Tyr Thr Gly Asn Pro Leu Ile Pro Trp Asp Tyr Cys
    450                 455                 460

Pro Ile Ser Arg Cys Glu Gly Asp Thr Thr Pro Thr Ile Val Asn Leu
465                 470                 475                 480

Asp His Pro Val Ile Ser Cys Ala Lys Thr Lys Gln Leu Arg Val Val

                485                 490                 495

Asn Gly Ile Pro Thr Arg Thr Asn Ile Gly Trp Met Val Ser Leu Arg
            500                 505                 510

Tyr Arg Asn Lys His Ile Cys Gly Gly Ser Leu Ile Lys Glu Ser Trp
        515                 520                 525

Val Leu Thr Ala Arg Gln Cys Phe Pro Ser Arg Asp Leu Lys Asp Tyr
    530                 535                 540

Glu Ala Trp Leu Gly Ile His Asp Val His Gly Arg Gly Asp Glu Lys
545                 550                 555                 560

Cys Lys Gln Val Leu Asn Val Ser Gln Leu Val Tyr Gly Pro Glu Gly
                565                 570                 575

Ser Asp Leu Val Leu Met Lys Leu Ala Arg Pro Ala Val Leu Asp Asp
            580                 585                 590

Phe Val Ser Thr Ile Asp Leu Pro Asn Tyr Gly Cys Thr Ile Pro Glu
        595                 600                 605

Lys Thr Ser Cys Ser Val Tyr Gly Trp Gly Tyr Thr Gly Leu Ile Asn
    610                 615                 620

Tyr Asp Gly Leu Leu Arg Val Ala His Leu Tyr Ile Met Gly Asn Glu
625                 630                 635                 640

Lys Cys Ser Gln His His Arg Gly Lys Val Thr Leu Asn Glu Ser Glu
                645                 650                 655

Ile Cys Ala Gly Ala Glu Lys Ile Gly Ser Gly Pro Cys Glu Gly Asp
            660                 665                 670

Tyr Gly Gly Pro Leu Val Cys Glu Gln His Lys Met Arg Met Val Leu
        675                 680                 685

Gly Val Ile Val Pro Gly Arg Gly Cys Ala Ile Pro Asn Arg Pro Gly
    690                 695                 700

Ile Phe Val Arg Val Ala Tyr Tyr Ala Lys Trp Ile His Lys Ile Ile
705                 710                 715                 720

Leu Thr Tyr Lys Val Pro Gln Ser
                725

<210>  7
<211>  8
<212>  PRT
<213>  Artificial

<220>
<223>  DYKDDDDK tag

<400>  7

Asp Tyr Lys Asp Asp Asp Asp Lys
1               5